# EUROPEAN PATENT SPECIFICATION

(11) **EP 1 594 548 B1**
(45) Date of publication and mention of the grant of the patent: **09.03.2011**
(21) Application number: 04703628.0
(22) Date of filing: 20.01.2004
(51) Int. Cl.: A61K 47/48

(54) **ANTHRACYCLINE-ANTI-CD74 ANTIBODY CONJUGATES**
ANTHRACYCLIN-ANTI-CD74-ANTIKÖRPERKONJUGATE
CONJUGUES D'ANTICORPS ANTI-CD74 ET D'ANTHRACYCLINE

(30) Priority: 24.01.2003 US 442125 P
(43) Date of publication of application: 16.11.2005
(73) Proprietor: Immunomedics, Inc., Morris Plains, NJ 07950 (US)
(72) Inventor: GRIFFITHS, Gary L., North Potomac, MD 20878 (US)
(74) Representative: Bohmann, Armin K.
(86) International application number: PCT/US2004/001367
(87) International publication number: WO 2004/067038

(56) References cited:
- EP-A- 0 476 408
- EP-A- 0 665 020
- WO-A-97/23243
- WO-A-03/074566
- WO-A-03/074567
- WO-A1-00/74718
- US-A- 5 521 290
- SCOTT W L ET AL: "Synthesis of reagents for the one step incorporation of hydrazide functionality onto the lysine residues of proteins, and their use as linkers for carbonyl containing molecules" BIOORGANIC & MEDICINAL CHEMISTRY LETTERS, OXFORD, GB, vol. 6, no. 13, 9 July 1996 (1996-07-09), pages 1491-1496, XP004175739 ISSN: 0960-894X
- LAU A.ET AL: "Novel doxorubicin-monoclonal anti-carcinoembryonic antigen antibody immunoconjugate activity in vitro" BIORGANIC AND MEDICINAL CHEMISTRY, vol. 3, no. 10, 1995, pages 1305-1312, XP001193825
- DALTON KING H. ET AL: "BR96 conjugates of highly potent anthracyclines" BIOORGANIC AND MEDICINAL CHEMISTRY LETTERS, vol. 13, 2003, pages 2119-2122, XP001193826
- GRIFFITHS G. L. ET AL: "Cure of SCID mice bearing human B-lymphoma xenografts by an anti CD74 antibody-anthracyclin conjugate" CLINICAL CANCER RESEARCH, vol. 9, 15 December 2003 (2003-12-15), pages 6567-6571, XP001189638
- GRIFFITHS G. L. ET AL: "Promising therapeutic activity of a new drug immunoconjugate, IMMU-110, in a Human Burkitt lymphoma model" BLOOD, vol. 102, no. 11, 16 November 2003 (2003-11-16), page 645A, XP001193747
- FROESCH B.A. ET AL: "Preparation and functional evaluation of new doxorubicin immunoconjugates containing an acid-sensitive linker on small-cell lung cancer cells" CANCER IMMUNOL IMMUNOTHER, vol. 42, no. 1, 1 January 1996 (1996-01-01), pages 55-63, XP002959259 SPRINGER-VERLAG, BERLIN, DE
- FROESCH B.A. ET AL.: "Combination of doxorubicin-immunoconjugates and molecular intervention in bcl-2 oncogene expression to overcome drug resistance in small cell lung cancer" PROCEEDINGS OF THE AMERICAN ASSOCIATION FOR CANCER RESEARCH ANNUAL MEETING, vol. 36, 1995, page 341, XP001536880

## Description

The present invention is related to a conjugate of an anthracycline drug and antibody, processes for producing such conjugate, the use of such conjugate for the manufacture of a medicament, the use of two or more conjugates of an antibody and an anthracycline drug, whereby one of the conjugates is the conjugate, and a kit comprising such conjugate.

### BACKGROUND OF THE INVENTION

For many years it has been a goal of scientists in the field of specifically targeted drug therapy that antibodies could be used for the specific delivery of chemotherapy drugs to human cancers. Realization of such a goal could finally bring to cancer chemotherapy the concept of the magic bullet. A significant advance toward achieving this goal came with the advent of the hybridoma technique of Köhler and Milstein in 1975, and the subsequent ability to generate monoclonal antibodies (mAbs). During the past 25 years mAbs have been raised against many antigenic targets that are over-expressed on cancerous cells. Either alone, or as conjugates of drugs, toxins, radionuclides or other therapy agents, many mAbs have been tested pre-clinically, and later in clinical trials. Generally, mAbs by themselves, often termed "naked mAbs," have not been successful at making long-term survivorship the norm in patients with solid tumors, although survival advantages have lately been seen with mAb treatments directed against both breast and colon cancer (mAbs against HER2-*neu* and 17-1A, respectively). With hematological malignancies more success is being achieved with naked mAbs, notably against the B-cell lymphomas (mAbs against CD20 and CD22 on the surface of B-cells).

It appears self evident, however, that the use of conjugates of tumor-associated mAbs and suitable toxic agents will be more efficacious than naked mAbs against most clinical cases of cancer. Here, a mAb also carries a toxic agent specifically to the diseased tissue, in addition to any toxicity it might inherently have by virtue of natural or reengineered effector functions provided by the Fc portion of the mAb, such as complement fixation and ADCC (antibody dependent cell cytotoxicity), which set mechanisms into action that may result in cell lysis. However, it is possible that the Fc portion is not required for therapeutic function, as in the case of mAb fragments, other mechanisms, such as apoptosis, inhibiting angiogenesis, inhibiting metastatic activity, and/or affecting tumor cell adhesion, may come into play. The toxic agent is most commonly a chemotherapy drug, a particle-emitting radionuclide, or a bacterial or plant toxin. Each type of conjugate has its own particular advantages. Penetrating radionuclides and the bacterial and plant toxins are extremely toxic, usually orders of magnitude more toxic than standard chemotherapy drugs. This makes the former two useful with mAbs, since in a clinical situation the uptake of mAbs into diseased tissue is extremely low. The low mAb tumor uptake in clinical practice and the relatively low toxicity profile of cancer chemotherapy drugs, combined, is a major reason why mAb-drug conjugates have failed to live up to their promise, to date.

In preclinical animal xenograft models, set up to study human cancer, many mAb conjugates have been described which are able to completely regress or even cure animals of their tumors. However, tumor uptakes of mAb conjugates in many of these animal xenograft models are often in the 10-50% injected dose per gram of tissue range, whereas in the clinical situation, tumor uptakes in the 0.1-0.0001 % injected dose per gram of tissue are more normal. It is no surprise, then, that mAb conjugates made with the more toxic radionuclides and toxins have generally fared somewhat better, clinically, than the corresponding mAb-drug conjugates with standard chemotherapeutic drugs. However, radionuclide mAb conjugates can often produce great toxicity due to the presence of a great excess of circulating, decaying radioactivity compared to tumor-localized activity. Toxin-mAb conjugates have suffered from the dual drawbacks of great non-target tissue damage and great immunoreactivity toward the plant or bacterial protein that is generally used. Whereas mAbs can now be made in human or in humanized (complementarity-determining region-grafted) forms, de-immunization of the toxin part of any conjugate will likely remain a significant obstacle to progress.

Despite the lack of necessary efficacy in a clinical setting seen to date, mAb-drug conjugates still have compelling theoretical advantages. The drug itself is structurally well defined, not present in isoforms, and can be linked to the mAb protein using very well defined conjugation chemistries, often at specific sites remote from the mAbs' antigen binding regions. MAb-drug conjugates can be made more reproducibly than chemical conjugates involving mAbs and toxins, and, as such, are more amenable to commercial development and regulatory approval. For such reasons, interest in drug conjugates of mAbs has continued despite the disappointments encountered. In some recent instances, however, preclinical results have been quite promising. With continuing refinements in conjugation chemistries, and the ability to remove or reduce immunogenic properties of the mAb, the elusive promise of useful mAb-drug conjugates for clinical cancer therapy are being newly considered.

Relevant early work on mAb-drug conjugates found during *in vitro* and *in vivo* preclinical testing that the chemical linkages used often resulted in the loss of a drug's potency. Thus, it was realized many years ago that a drug would ideally need to be released in its original form, once internalized by a target cell by the mAb component, in order to be a useful therapeutic. Work during the 1980s and early 1990s then focused largely on the nature of the chemical linker between the drug and the mAb. Notably, conjugates prepared using mild acid-cleavable linkers were developed, based on the observation that pH inside tumors was often lower than normal physiological pH (U.S. Patent Nos. 4,542,225; 4,569,789; 4,618,492; and 4,952,394). This approach culminated in a landmark paper by Trail et al. (Science 261:212-215 (1993)) showing that mAb-doxorubicin (DOX) conjugates, prepared with appropriate linkers, could be used to cure mice bearing a variety of human tumor xenografts, in preclinical studies. This promising result was achieved with an antibody (termed BR96) that had a very large number of receptors on the tumor cells being targeted, the mAb-drug conjugate was highly substituted (6-8 DOX residues per unit of mAb), and the conjugate was given in massive doses on a repeat basis.

In the clinical situation, tumor uptakes of mAbs would be much lower, and since this variable was something that had to be addressed, more toxic drugs, would be needed to achieve a desirable therapeutic effect. More toxic drugs were used in the development of several distinct mAb-drug conjugates (U.S. Patent Nos. 5,208,020; 5,416,064; 5,877,296; and 6,015,562). These efforts use drugs, such as derivatives of maytansinoids and calicheamicin, which are essentially too toxic to be used in standard chemotherapy. Conjugation to a mAb enables relatively more of the drug to be targeted to a tumor in relation to the often non-specific cell and protein binding seen with chemotherapy alone. The exquisite toxicity of drugs such as these might overcome the low levels of tumor-targeted mAb seen clinically, due to the low level of antigen binding sites generally seen on tumor targets. In preclinical studies, cures of mice bearing human tumor xenografts were seen at much lower doses of mAb-drug conjugate, than seen previously with mAb-drug conjugates using standard drugs, such as DOX (Liu et al., Proc. Natl. Acad. Sci. USA 93:8616-8623 (1996) and Hinman et al., Cancer Res. 53:3336-3342 (1993)). In the case of the maytansinoid-mAb conjugates (Liu), the amount of conjugate needed for therapy was over > 50-fold less than needed previously with DOX conjugates (Trail, *supra*).

During development of these conjugates the linker between drug and mAb was thought to be critical for retention of good anti-tumor activity both *in vitro* and *in* vivo. The cited conjugates were made with an intracellularly-cleavable moiety (hydrazone) and a reductively labile (disulfide) bond between the drug and the mAb. While the hydrazone bond is apparently stable to *in vivo* serum conditions, normal disulfide bonds were found to be not stable enough for practical use. Conjugates were made that replaced a standard disulfide linkage with a hindered (geminal dimethyl) disulfide linkage in the case of the calicheamicins, or a methyl disulfide in the case of the maytansinoids. While this work was being done, separate work also continued on newer anthracycline-substituted mAb conjugates. In the case of newer DOX conjugated mAbs, it was found that superior results could be obtained by incorporating just a hydrazone as a cleavable unit, and attaching DOX to mAb via a thioether group, instead of a disulfide (U.S. Patent No. 5,708,146). When linked in such a manner, and also using a branched linker capable of doubling the number of DOX units per MAb substitution site, an approximate order of magnitude increase in the efficacy of the new DOX-MAb conjugates were obtained (King et al., Bioconjugate Chem. 10:279-288, (1999)).

### SUMMARY OF THE INVENTION

The problem underlying the present invention is solved by the subject matter of the independent claims. Preferred embodiments may be taken from the dependent claims.

The present invention is thus directed to new internalizing antibody conjugates of anthracycline drugs wherein said anthracycline drug and said antibody are linked via a linker comprising a hydrazide and a maleimide, and said antibody is an internalizing antibody directed against CD74 and wherein the antibody-anthracycline conjugate is internalized into target cells. Specific embodiments are exemplified by conjugates of doxorubicin (DOX), epirubicin, morpholinodoxorubicin (morpholino-DOX), cyanomorpholinodoxorubicin (cyanomorpholino-DOX), and 2-pyrrolino-doxorubicin (2-PDOX). 2-PDOX is particularly toxic, incorporating an enamine in its structure, which can act not only as an intercalator and topoisomerase inhibitor, but also as an alkylating agent having increased toxicity. Like DOX, 2-PDOX has relatively good aqueous solubility which means that it can be coupled to mAbs in multiply substituted amounts without precipitation of the mAb. The drugs described in detail below are consistently substituted at an average of 8 (typically measured at 7 - 9) drug moieties per molecule of mAb. The number of drugs, however, may also range between 6 to 10 molecules per molecule of mAb.

In one aspect, the invention relates to such immunoconjugate comprising a targeting moiety, an anthracycline drug and a linker binding the targeting moiety via a thiol group and the anthracycline chemotherapeutic drug via an intracellularly-cleavable moiety.

In a preferred embodiment of the present invention, the targeting moiety is a mAb, the anthracycline chemotherapeutic drug is DOX, 2-PDOX, morpholino-DOX and morpholinocyano-DOX, and the intracellularly-cleavable moiety is a hydrazone.

In another embodiment, the invention relates to an immunoconjugate comprising a disease-targeting antibody and an anthracycline chemotherapeutic drug. Many hundreds of examples of anthracycline drugs have been synthesized over the last 30-40 years or so, and they are discussed in detail elsewhere (*see:* Anthracycline Antibiotics; New Analogs, methods of Delivery, and Mechanisms of Action, Waldemar Priebe, Editor, ACS Symposium Series 574, American Chemical Society, Washington DC, 1994). Such analogs are envisaged as within the scope of the current invention

In a preferred embodiment, the invention relates to an immunoconjugate comprising a disease-targeting antibody and an anthracycline chemotherapeutic drug of the formulae I and II: 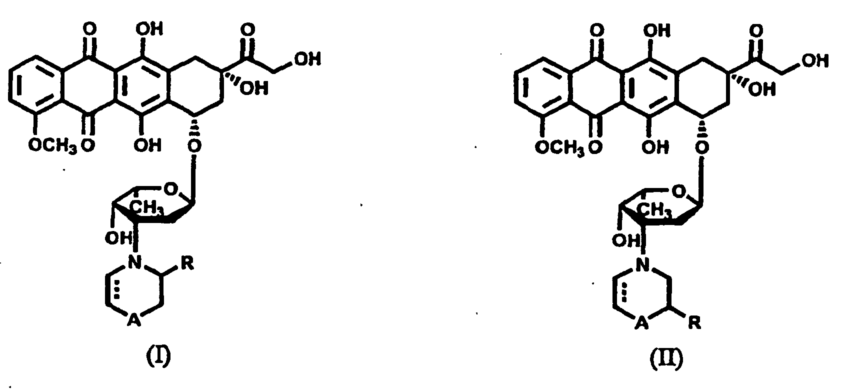 wherein, A is nothing or it may be selected from the group consisting of NH, N-alkyl, N-cycloalkyl, O, S, and CH₂; the dotted line denotes a single or a double bond; and R is H or CN; and a linker binding the targeting moiety via a sulfide group and the anthracycline chemotherapeutic drug via an intracellularly cleavable moiety. When A is "nothing," the carbon atoms adjacent to A, on each side, are connected by a single bond, thus giving a five membered ring.

As used herein, "alkyl" refers to a saturated aliphatic hydrocarbon radical including straight chain and branched chain groups of 1 to 20 carbon atoms (whenever a numerical range; e.g. "1-20", is stated herein, it means that the group, in this case the alkyl group, may contain 1 carbon atom, 2 carbon atoms, 3 carbon atoms, etc. up to and including z 20 carbon atoms). Alkyl groups containing from 1 to 4 carbon atoms are referred to as lower alkyl groups. More preferably, an alkyl group is a medium size alkyl having 1 to 10 carbon atoms e.g., methyl, ethyl, propyl, 2-propyl, n-butyl, iso-butyl, tert-butyl, pentyl. Most preferably, it is a lower alkyl having 1 to 4. carbon atoms e.g., methyl, ethyl, propyl, 2-propyl, n-butyl, iso-butyl, or tert-butyl.

As used herein "cycloalkyl" refers to a 3 to 8 member all-carbon monocyclic ring, an all-carbon 5-member/6-member or 6-member/6-member fused bicyclic ring or a multicyclic fused ring (a "fused" ring system means that each ring in the system shares an adjacent pair of carbon atoms with each other ring in the system) group wherein one or more of the rings may contain one or more double bonds but none of the rings has a completely conjugated pi-electron system. Examples, without limitation, of cycloalkyl groups are cyclopropane, cyclobutane, cyclopentane, cyclopentene, cyclohexane, cyclohexadiene, adamantane, cycloheptane, cycloheptatriene. A cycloalkyl group may be substituted or unsubstituted.

In another preferred embodiment, the intracellularly cleavable moiety is a hydrazone.

It is disclosed that the mAb is a mAb that targets tumor-associated antigens. Tumor-associated antigens are defined as antigens expressed by tumor cells, or their vasculature, in a higher amount than in normal cells, wherein the normal cells are vital to cellular functions essential for the patient to survive. Tumor-associated antigens may also be antigens associated with different normal cells, such as lineage antigens in hematopoietic cells, B-cells, T-cells or myeloid cells, whereby a patient can survive with a transient, selective decrease of said normal cells, while the malignant cells expressing the same antigen(s) are sufficiently destroyed to relieve the patient of symptoms and also improve the patient's condition. The mAb may also be reactive with an antigen associated with hematologic malignancies

It is disclosed that the mAb is selected from the group of B-cell, T-cell, myeloid-cell, and other hematopoietic cell-associated antigens, such as CD19, CD20, CD21, CD22, CD23 in B-cells; CD33, CD45, and CD66 in myeloid cells; IL-2 (TAC or CD25) in T-cells; MUC1, tenascin, CD74, HLA-DR, CD80 in diverse hematopoietic tumor types; CEA, CSAp, MUC1, MUC2, MUC3, MUC4, PAM4, EGP-1, EGP-2, AFP, HCG, HFR2/neu, EGFR, VEGF, PlGF, Le(y), carbonic anhydrase IX, PAP, PSMA, MAGE, S100, tenascin, and TAG-72 in various carcinomas, tenascin in gliomas, and antigens expressed by the vasculature and endothelial cells, as well as the supportive stroma, of certain tumors. In still another preferred embodiment, the mAb is LL1 (anti-CD74). Also disclosed are LL2 (anti-CD22), hA20 and rituximab (anti-CD20), M195 (anti-CD33), RS7 (anti-epithelial glycoprotein-1 (EGP-1)), 17-1A (anti-EGP-2), PAM-4, BrE3, and KC4 (all anti-MUC1), MN-14 (anti-carcinoembryonic antigen (CEA)), Mu-9 (anti-colon-specific antigen-p), Immu 31 (an anti-alpha-fetoprotein), anti-TAG-72 (e.g., CC49) anti-Tn, J591 (anti-PSMA), BC-2 (an anti-tenascin antibody) and G250 (an anti-carbonic anhydrase IX mAb). Other useful antigens that may be targeted using conjugates include HER-2/*neu*, CD19, CD20 (e.g., C2B8, hA20, cA20, IF5 Mabs) CD21, CD23, CD33, CD40, CD80, alpha-fetoprotein (AFP), VEGF, EGF receptor, PlGF (placenta growth factor), ILGF-1 (insulin-like growth factor-1), MUC1, MUC2, MUC3, MUC4, PSMA, gangliosides, HCG, EGP-2 (e.g., 17-1A), CD37, HLA-DR, CD30, Ia, A3, A33, Ep-CAM, KS-1, Le(y), S100, PSA, tenascin, folate receptor, Thomas-Friedenreich antigens, tumor necrosis antigens, tumor angiogenesis antigens, Ga 733, IL-2 (CD25), T101, MAGE, CD66, CEA, NCA95, NCA90 or a combination thereof. An antigen which may be targeted using the conjugate of the invention is Ii. An antigen which may be targeted using the conjugate of the invention is Ii.

In an especially preferred embodiment the targeting mAb is directed against the CD74 antigen which is then rapidly internalized with the antibody.

In yet another preferred embodiment, the linker is a 4-[N-maleimidomethyl]cyclohexane-1-carboxylhydrazide radical.

Also described are processes for the preparation of the compositions of the invention, together with methods-of-use of the said compositions as subject to the attached claims.

### BRIEF DESCRIPTION OF THE DRAWINGS

FIG 1 is a representative size-exclusion HPLC trace of an anthracycline-antibody conjugate prepared using the methods described.

FIG 2 illustrates the *in vitro* efficacy of the DOX-LL1 conjugate against Burkitt lymphoma Raji cells, versus a DOX conjugate of the non-targeting MN-14 antibody at a concentration of drug-mAb conjugate of 1 µg/mL. The DOX-LL1 conjugate shows a three-order of magnitude difference in the fraction of surviving cells, in comparison to the DOX-MN-14 conjugate.

FIG 3 is illustrates the efficacy of a single 100 µg dose of 2-PDOX-RS7 conjugate in the DU145 prostate xenograft model in nude mice.

FIG 4 illustrates the efficacy of single doses of 2-PDOX- and DOX-conjugates of the LL1 antibody in the aggressive RAJI/SCID mouse systemic tumor model. Animals were injected i.v. with Raji B-cell lymphoma cells, and treated five days later with the conjugates designated in the figure.

FIG 5 illustrates the efficacy of a single dose of 2-PDOX-LL1 antibody in the aggressive RAJI/SCID mouse systemic tumor model, compared to untreated controls given no conjugate, or a group of animals given the non-targeting control conjugate, 2-PDOX-MN-14.

### DETAILED DESCRIPTION OF THE PREFERRED EMBODIMENTS

As used herein, "a" or "an" means "one or more" unless otherwise specified.

### Introduction

Chemotherapeutic drugs, such as those discussed above, can be coupled to antibodies by several methods to form a mAb-drug conjugate. For example, the chemotherapeutic drugs may be attached to the mAb, or fragments thereof, after reduction of the mAb inter-chain disulfide bonds. This approach generates an average of eight-to-ten (depending on IgG type) free thiol groups per molecule of antibody, and does so in a reproducible manner at the limiting levels of thiol used in the reduction reaction. This method of attachment of the chemotherapeutic drugs is advantageous for the following reasons: first, the attached chemotherapeutic drugs are placed in an internal or semi-internal site on the mAb, or fragments thereof, which is not exposed on hydrophilic lysine residues. This serves to keep them more stable due to the more hydrophobic areas of the mAb, where the chemotherapeutic drugs are placed. Second, such a site does not alter the overall charge of the mAb, or fragments thereof. Third, placement on internal thiols is less likely to interfere in the ADCC and complement actions that are particularly important when naked versions of the mAb are used. Thus, the attachment site is chosen to be non-interfering, such that ADCC and complement fixation, can be complementary to the mAbs, or the mAb fragments, role as a drug delivery vehicle. Fourth, placement at the internal thiol positions is less likely to lead to an immune response to the chemotherapeutic drugs, compared to placement of a multitude of chemotherapeutic drugs molecules on exposed lysine groups. In some-embodiments, the overall electric charge of the antibody in the Ab-drug conjugate is not changed as compared to the charge of the antibody prior to the coupling. This is because no lysine residues are used in the conjugation reaction, and therefore no free, positive amino groups are modified to form, for example, neutral amide bonds.

### Antibodies

An antibody, as described herein, refers to a full-length (i.e., naturally occurring or formed by normal immunoglobulin gene fragment recombinatorial processes) immunoglobulin molecule (e.g., an IgG antibody) or an immunologically active (i.e., specifically binding) portion of an immunoglobulin molecule, like an antibody fragment.

An antibody fragment is a portion of an antibody such as F(ab')₂, F(ab)₂, Fab', Fab, Fv, scFv (single chain Fv). Regardless of structure, an antibody fragment binds with the same antigen that is recognized by the intact antibody and it therefore, an antigen-binding fragment of the antibody of which it is a portion.

The term "antibody fragment" also includes any synthetic or genetically engineered protein that acts like an antibody by binding to a specific antigen to form a complex. For example, antibody fragments include isolated fragments consisting of the variable regions, such as the "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region. The Fv fragments may be constructed in different ways as to yield multivalent and/or multispecific binding forms. Multivalent binding forms react with more than one binding site against the specific epitope, whereas multispecific forms bind more than one epitope (either of the antigen or even against the specific antigen and a different antigen).

As used herein, the term antibody fusion protein is a recombinantly-produced antigen-binding molecule in which two or more of the same or different natural antibody, single-chain antibody or antibody fragment segments with the same or different specificities are linked. A fusion protein comprises at least one specific binding site.

Valency of the fusion protein indicates the total number of binding arms or sites the fusion protein has to antigen(s) or epitope(s); i.e., monovalent, bivalent, trivalent or mutlivalent. The multivalency of the antibody fusion protein means that it can take advantage of multiple interactions in binding to an antigen, thus increasing the avidity of binding to the antigen, or to different antigens. Specificity indicates how many different types of antigen or epitope an antibody fusion protein is able to bind; i.e., monospecific, bispecific, trispecific, multispecific. Using these definitions, a natural antibody, e.g., an IgG, is bivalent because it has two binding arms but is monospecific because it binds to one type of antigen or epitope. A monospecific, multivalent fusion protein has more than one binding site for the same antigen or epitope. For example, a monospecific diabody is a fusion protein with two binding sites reactive with the same antigen. The fusion protein may comprise a multivalent or multispecific combination of different antibody components or multiple copies of the same antibody component.

In a preferred embodiment of the present invention, antibodies, such as monoclonal antibodies (mAbs), are used that recognize or bind to markers or tumor-associated antigens that are expressed at high levels on target cells and that are expressed predominantly or only on diseased cells versus normal tissues, and antibodies that internalize rapidly. An Antibody useful within the scope of the present invention includes LL1 (anti-CD74). Also disclosed are antibodies against tumor-associated antigens, such as antibodies with properties as described above (and show distinguishing properties of different levels of internalization into cells and microorganisms), and contemplate the use of in cancer, the following mAbs: LL2 (anti-CD22), M195 (anti-CD33), MN3 (anti-NCA90), RS7 (anti-epithelial glycoprotein-1(EGP-1)), PAM-4, BrE3 and KC4 (all anti-MUC1), MN-14 (anti-carcinoembryonic antigen (CEA)), Mu-9 (anti-colon-specific antigen-p), Immu 31 (an anti-alpha-fetoprotein), anti-TAG-72 (e.g., CC49), anti-Tn, J591 (anti-PSMA), M195 (anti-CD33) and G250 (an anti-carbonic anhydrase IX mAb). Other useful antigens and different epitopes of such antigens that may be targeted using conjugates include HER-2/neu, , CD19, CD20 (e.g., C2B8, hA20, 1F5 Mabs) CD21, CD23, CD25, CD30, CD33, CD37, CD40, CD80, alpha-fetoprotein (AFP), VEGF, EGF receptor, PlGF, MUC1, MUC2, MUC3, MUC4, PSMA, PAP, carbonic anhydrase IX, TAG-72, GD2, GD3, , HCG, EGP-2 (e.g., 17-1A),, HLA-DR, CD30, Ia, A3, A33, Ep-CAM, KS-1, Le(y), S100, PSA, tenascin, folate receptor, Tn or Thomas-Friedenreich antigens, tumor necrosis antigens, tumor angiogenesis antigens, Ga 733, , T101, MAGE, or a combination thereof. A number of the aforementioned antigens are disclosed in U.S. Provisional Application Serial No. 60/426,379, entitled "Use of Multi-specific, Non-covalent Complexes for Targeted Delivery of Therapeutics," filed November 15, 2002.

In another preferred embodiment of the present invention, antibodies are used that internalize rapidly and are then re-expressed on cell surfaces, enabling continual uptake and accretion of circulating-antibody-chemotherapeutic drug conjugate by the cell wherein the drug is anthracycline and the antibody-anthracyline conjugate is internalized into target cells and then re-expressed on the cell surface. An example of a most-preferred antibody/antigen pair is LL1 and CD74 (invariant chain, class It-specific chaperone, Ii). The C74 antigen is highly expressed on B cell lymphomas, certain T cell lymphomas, melanomas and certain other cancers (Ong et al., Immunology 98:296-302 (1999)).

In a preferred embodiment the antibodies that are used in the treatment of human disease are human or humanized (CDR-grafted into a human framework) versions of antibodies; although murine, chimeric and primatized versions of antibodies can be used. For veterinary uses, the same-species IgG would likely be the most effective vector, although cross-species IgGs would remain useful, such as use of murine antibodies in dogs (e.g., L243 anti-HLA-DR mAb for treating canine lymphoma). Same species immunoglobulin (IgG)s molecules as delivery agents are mostly preferred to minimize immune responses. This is particularly important when considering repeat treatments. For humans, a human or humanized IgG antibody is less likely to generate an anti-IgG immune response from patients. Targeting an internalizing antigen, antibodies such as hLL1 and hLL2 rapidly internalize after binding to target cells, which means that the conjugated chemotherapeutic drug is rapidly internalized into cells.

An immunomodulator such as a cytokine can also be conjugated to the monoclonal antibody-anthracycline drug of the invention, or can be administered unconjugated to the chimeric, humanized or human monoclonal antibody-anthracycline drug conjugate of the preferred embodiments of the present invention. The immunomodulator can be administered before, concurrently or after administration of the monoclonal antibody-anthracyline drug conjugate of the preferred embodiments of the present invention. The immunomodulator can also be conjugated to a hybrid antibody consisting of one or more antibodies binding to different antigens :wherein one such antigen is CD74. Such an antigen may also be an immunomodulator. For example, CD40 or other immunomodulators can be administered in combination with anti-CSAp or anti-CSAp/non-CSAp antibody combination either together, before or after the antibody combinations are administered. The monoclonal antibody-anthracyline drug conjugate of the invention can also be used in combination with, or conjugated to, as a fusion protein, such as against CD40.

As used herein, the term "immunomodulator" includes cytokines, stem cell growth factors, lymphotoxins, such as tumor necrosis factor (TNF), and hematopoietic factors, such as interleukins (e.g., interleukin-1 (IL-1), IL-2, IL-3, IL-6, IL-10, IL-12, IL-18, and IL-21), colony stimulating factors (e.g., granulocyte-colony stimulating factor (G-CSF) and granulocyte macrophage-colony stimulating factor (GM-CSF)), interferons (e.g., interferons-α, -β and -γ), the stem cell growth factor designated "S1 factor," erythropoietin and thrombopoietin. Examples of suitable immunomodulator moieties include IL-2, IL-6, IL-10, IL-12, IL-18, IL-21, interferon-γ, TNF-α.

An immunomodulator is a therapeutic agent as defined in the present invention that when present, alters, suppresses or stimulates the body's immune system. Typically, the immunomodulator useful in the present invention stimulates immune cells to proliferate or become activated in an immune response cascade, such as macrophages, B-cells, and/or T-cells. An example of an immunomodulator as described herein is a cytokine, which is a soluble small protein of approximately 5-20 kDs that are released by one cell population (e.g., primed T-lymphocytes) on contact with specific antigens, and which act as intercellular mediators between cells. As the skilled artisan will understand, examples of cytokines include lymphokines, monokines, interleukins, and several related signalling molecules, such as tumor necrosis factor (TNF) and interferons. Chemokines are a subset of cytokines. Certain interleukins and interferons are examples of cytokines that stimulate T cell or other immune cell proliferation.

In a preferred embodiment of the present invention, the immunomodulator enhances the effectiveness of the anthracycline drug-antibody conjugate, and in some instances by stimulator effector cells of the host.

### Antibody-chemotherapeutic drug conjugates

The present invention is directed to a conjugate of an anthracycline drug and an antibody, wherein the anthracycline drug and the antibody are linked via a linker comprising a hydrazide and a maleimide as defined in claim 1. The linker preferably is 4-(N-maleimidomethyl)cyclohexane-1-carboxyl hydrazide. The conjugate preferably has the formula: 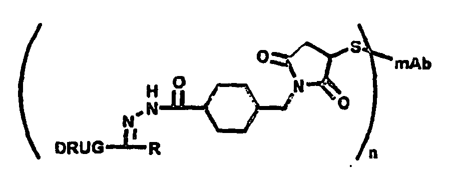 wherein n is 6 to 10.

Further, the antibody is directed against or recognizes CD74. The antibody may be a monoclonal antibody, an antigen-binding fragment thereof or an antibody fusion protein. The antibody fusion protein may be multivalent and/or multispecific. The antibody fusion protein in the conjugate may comprise two or more of the same or different natural or synthetic antibody, single-chain antibody or antibody fragment segments with the same or different specificities. The antibody or antibody fragment of the fusion protein can be selected from the group consisting of LL1 Also disclosed is that the antibody or antibody fragment of the fusion protein can be selected from the group consisting of LL2, M195, MN-3, RS7, 17-2-1A, RS11, PAM-4, KC4, BrE3, MN-14, Mu-9, Immu 31, CC49" Tn antibody, J591, Le(y) antibody and G250.

This tumor-associated antigen may be targeted by an internalizing antibody. The conjugate is useful for targeting carcinomas, sarcomas, lymphomas, leukemias, gliomas or skin cancers, such as melanomas. The tumor-associated antigen preferably is selected from the group consisting of CD74 and li. Also diclosed are, CD22, EPG-1, CEA, colon-specific antigen-p mucin (CSAp), carbonic anhydrase IX, HER-2/*neu*,, CD19, CD20, CD21, CD23, CD25, CD30, CD33, CD40, CD45, CD66, NCA90, NCA95, CD80, alpha-fetoprotein (AFP), VEGF, EGF receptor, PlGF, MUC1, MUC2, MUC3, MUC4, PSMA, GD2, GD3 gangliosides, HCG, EGP-2, CD37, HLA-D-DR, CD30, Ia, Ii, A3, A33, Ep-CAM, KS-1, Le(y), S100, PSA, tenascin, folate receptor, Tn and Thomas-Friedenreich antigens, tumor necrosis antigens, tumor angiogenesis antigens, Ga 733, IL-2,, MAGE, and a combination thereof More preferably the tumor-associated antigen is selected from the group consisting of CD74, CD19, CD20, CD22, CD33, EPG-1, MUC1, CEA and AFP. These tumor-associated antigens may be lineage antigens (CDs) of B-cells, T-cells, myeloid cells, or antigens associated with hematologic malignancies.

The antibody portion of the conjugate can be murine, chimeric, primatized, humanized, or human. The antibody may be an intact immunoglobulin or an antigen-binding fragment thereof, such as an IgG or a fragment thereof Preferably, the antibody is directed against B-cells, such as an antigen selected from the group consisting of CD74. Also diclosed are CD19, CD20, CD21, CD22, CD23, CD30, CD37, CD40, CD52, CD80, and HLA-DR. The antibody, antigen-binding fragment thereof or fusion protein, preferably is selected from the group of LL1. Also disclosed are LL2, L243, C2B8, A20, MN-3, M195, MN-14, anti-AFP, Mu-9, PAM-4, RS7, RS11 and 17-1A. More preferably, the antibody is LL1. Also disclosed are LL2, L243, C2B8, or hA20. Additionally, the antibody is linked to the drug via a linker which is attached to a reduced disulfide bond on the antibody, which may be an interchain disulfide bond on the antibody.

The anthracycline drug portion of the conjugate is selected from the group consisting of daunorubicin, doxorubicin, epirubicin, 2-pyrrolinodoxorubicin, morpholino-doxorubicin, and cyanomorpholino-doxorubicin. Further, the anthracycline drug can be linked to the antibody through the 13-keto moiety. Preferably, there are 6 - 10 molecules of anthracycline drug per molecule of antibody. Additionally, the antibody-anthracycline conjugate is internalized into target cells, and the antigen is then re-expressed on the cell surface.

The present invention is directed to a process for producing the conjugate described herein, wherein the linker is first conjugated to the anthracycline drug, thereby producing an anthracycline drug-linker conjugate, and wherein the anthracycline drug-linker conjugate is subsequently conjugated to a thiol-reduced monoclonal antibody or antibody fragment. The anthracycline drug-linker conjugate may be purified prior to conjugation to the thiol-reduced monoclonal antibody or antibody fragment but it is not necessary to do so. Thus, preferably there is no need to purify the anthracycline drug-linker conjugate prior to conjugation to the thiol-reduced monoclonal antibody or antibody fragment. The process for preparing the conjugate should be such that the secondary reactive functional groups on the anthracycline drug are not compromised. Additionally, the process for preparing the conjugate should not compromise the alkylating groups on the anthracycline drugs. The anthracycline drug in the conjugate preferably is 2-pyrrolino-doxorubicin, morpholino-doxorubicin or cyanomorpholino-doxorubicin.

The chemotherapeutic drug molecules are separately activated for conjugation to the antibody such that they contain a free maleimide group, specific for thiol reaction at neutral pH. When the chemotherapeutic drug bears a reactive ketone, the ketone can be converted to hydrazone using the commercially available linker 4-[N-maleimidomethyl]cyclohexane-1-carboxylhydrazide (M₂C₂H; Pierce Chemical Co., Rockford, IL) [also supplied as the trifluoroacetate salt by Molecular Biosciences, Inc., Boulder, CO] as shown in Scheme I, below.

In Scheme I, the DRUG is a chemotherapeutic drug, preferably an anthracycline drug and the R group is either a hydrogen atom or a C₁-C₆ alkyl group optionally substituted with a hydroxyl group (-OH).

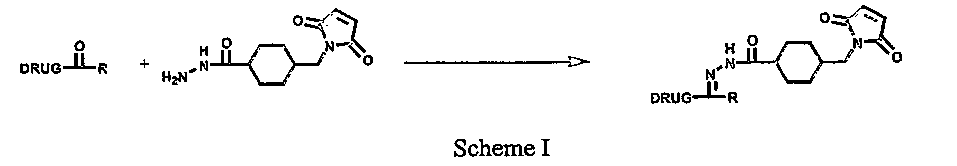

While not being bound by theory, the linker M₂C₂H is thought to be a particularlyuseful linker in the context of the preferred embodiments of the present invention for two reasons. First, the cyclohexyl group in the linker is thought to stabilize the hydrazone functionality. It is important that the hydrazone linkage used is substantially stable to serum conditions, and the cyclohexyl group proximal to the formed hydrazone results in a more stable hydrazone bond in comparison to a more standard straight-chain alkyl group. Second, the hydrazone produced from the reaction of the ketone with this carboxylhydrazide is cleaved once the chemotherapeutic drug-mAb conjugate is internalized into the cell.

The maleimide-substituted chemotherapeutic drugs, in slight excess (1 to 5 fold molar) to available thiol groups on the reduced mAb are mixed in an aqueous solution with the reduced mAb. The reaction is performed at neutral, near-neutral or below neutral pH, preferably from about pH 4 to about pH 7. The components are allowed to react for a short reaction time of from about 5 to about 30 minutes. The skilled artisan would recognize, however, that the reaction conditions may be optimized with respect to reaction time and pH. The chemotherapeutic drug-mAb conjugate, shown schematically below (wherein n is an integer from 1 to 10, preferably from 1 to 8), is then separated from chemotherapeutic drug and other buffer components by chromatography through size-exclusion and hydrophobic interaction chromatography columns. In a preferred embodiment, the drug is an anthracycline and n is an integer from 6 - 10.

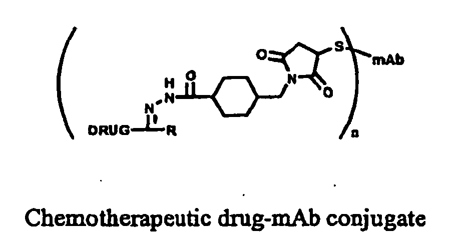

The above conditions are optimal in the case of 2-PDOX. The reaction conditions are optimal since they ensure that only the freely generated thiol groups of the mAb react with the maleimide-activated chemotherapeutic drug, while the enamine of 2-PDOX is not impinged by the reaction conditions. It is surprising that the thiol-maleimide coupling can be carried out in the presence of an alkylatable group, as exemplified here by the enamine group.

In accordance with the present invention, the chemotherapeutic drugs that are used are anthracycline drugs. These drugs comprise a large class of derivatives typified by one of the original members of the group, doxorubicin (DOX, shown below), and its isomer, epirubicin.

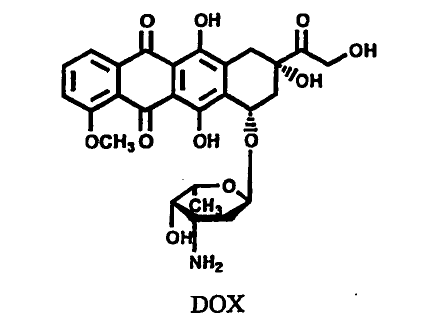

Both doxorubicin and epirubicin are widely used in cancer therapy. In another preferred embodiment of the present invention the chemotherapeutic drugs include analogs of the highly toxic 2-PDOX, namely, morpholino- and cyanomorpholino-doxorubicin (morpholino-DOX and cyanomorpholino DOX, respectively). In another embodiment the chemotherapeutic drugs include daunorubicin.

The skilled artisan will recognize that the anthracycline drugs of the embodiments of the present invention contain a number of reactive groups, which may be referred go as secondary reactive functional groups, that may require protection with protective groups well known in the art prior to conjugation of the drug with the linker and/or prior to conjugation of the drug-linker conjugate and the mAb; protection may be necessary so as to not compromise the integrity of the reactive groups. *See* Greene and Wuts, Protective Groups in Organic Synthesis (John Wiley & Sons 2d ed. 1991. The reactive groups include the carbonyl groups in the anthraquinone core of the anthracycline drugs; groups which, under certain conditions, may be react with a nucleophile. Other reactive groups include the various alcohol groups that are located throughout the anthracycline drug molecules; groups, which under certain conditions may react with electrophiles. Lastly, other reactive groups include the amine group present in DOX and the enamine group in 2-PDOX; both of which may react with an electrophile. In the case of anthracycline drugs bearing an alkylating group (e.g., the enamine of 2-PDOX), it may be necessary to control the reaction conditions such that the integrity of the alkylating group is not compromised.

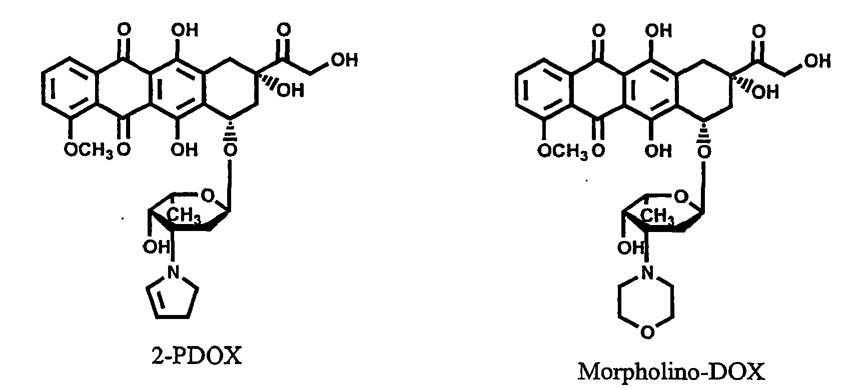

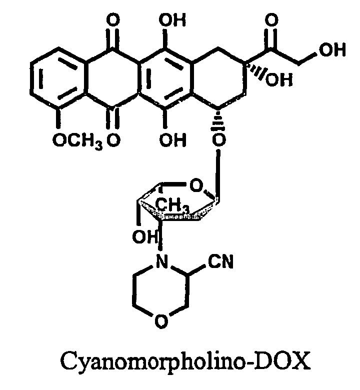

Within the anthracycline drug class, individual drugs, of toxicities varying over a 1-10,000 fold range (3-4 order-of-magnitude) range, can be interchanged on the basis of their varying toxicities, in order to generate more or less toxic immunoconjugates. Anthracyclines can exert their toxic effect on target cells by several mechanisms, including inhibition of DNA topoisomerase 2 (top 2), intercalation into DNA, redox reactions and binding to certain intracellular or membrane proteins. Additionally, analogs can be designed that have additional mechanisms of cell killing, such as a potential to be alkylated. Exemplary analogs are anthracylines bearing an alkylating moiety, as in the case of the 2-PDOX analog. In this instance, the alkylating moiety is an enamine group. In the 2-PDOX analog, the enamine group in the pyrrolino- ring is highly reactive to nucleophiles under physiologic conditions.

### Pharmaceutical Compositions and Methods of Administrations

Some embodiments of the present invention relate to a pharmaceutical composition comprising the mAb-drug conjugate of the present invention and a pharmaceutically acceptable carrier or excipient. By "pharmaceutically acceptable carrier" is intended a non-toxic solid, semisolid or liquid filler, diluent, encapsulating material or formulation auxiliary of any type known to persons skilled in the art. Diluents, such as polyols, polyethylene glycol and dextrans, may be used to increase the biological half-life of the conjugate.

The present invention also is directed to the conjugate of the invention for use in a method for treating disease in a mammal comprising administering the conjugate as described herin. The method also comprises administering the antibody-anthracycline conjugate described herein in all of it permutations preceded by, concomitantly with, or subsequent to other standard therapies, wherein said standard therapy is selected from the group consisting of radiotherapy, surgery and chemotherapy.

The present invention is intended to encompass the conjugate of the invention for use in a method for treating disease in a mammal comprising administering two or more conjugates of an antibody and an anthracycline drug that target different antigens or different epitopes of the same antigen on the same diseased cells wherein one conjugate is the conjugate of the invention. Additionally the present invention is intended to encompass the conjugate of the invention for use in a method for treating disease in a mammal comprising administering the conjugate of the invention preceded by, concomitantly with, or subsequent to a second antibody-based treatment, such that the second antibody in the second antibody-based treatment targets a different antigen or a different epitope on the same antigen on diseased cells than the antibody in the conjugate.

In some embodiments, the mAb-drug conjugate alone or a pharmaceutical composition comprising the mAb-drug conjugate of the present invention and a pharmaceutically acceptable carrier or excipient may be used in a method of treating a subject, comprising administering a therapeutically effective amount of the mAb-drug conjugate of the present invention to a subject.

In preferred embodiments, the subject is a mammal. Exemplary mammals include human, pig, sheep, goat, horse, mouse, dog, cat, cow, etc. Diseases that may be treated with the mAb-drug conjugate of the present invention include cancer, such as cancer of the skin, head and neck, lung, breast, prostate, ovaries, endometrium, cervix, colon, rectum, bladder, brain, stomach, pancreas, lymphatic system may be treated. Patients suffering from B- or T-cell cancer, non-Hodgkin's lymphoma, Hodgkin's disease, lymphatic or myeloid leukemias, multiple myeloma, sarcoma and melanoma may be treated by administration of a therapeutic amount of the mAb-drug conjugate of the present invention.

The mAb-drug conjugate of the present invention may be administered intravenously, intra-peritoneally, intra-arterially, intra-thecally, intra-vesically, or intratumorally. The conjugate may be given as a bolus or as an infusion on a repeat and/or a cyclical basis. The infusion may be repeated for one or more times depending on the dose of drug and tolerability of the conjugate in terms of side effects and is determined by the managing physician. One of ordinary skill will appreciate that effective amounts of the mAb-drug conjugate of the invention can be determined empirically. The agents can be administered to a subject, in need of treatment of cancer, as pharmaceutical compositions in combination with one or more pharmaceutically acceptable excipients. It will be understood that, when administered to a human patient, the total daily usage of the agents or composition of the present invention will be decided by the attending physician within the scope of sound medical judgement. The specific therapeutically effective dose level for any particular patient will depend upon a variety of factors: the type and degree of the cellular response to be achieved; activity of the specific mAb-drug conjugate or composition employed; the specific mAb-drug conjugate or composition employed; the age, body weight, general health, sex and diet of the patient; the time of administration, route of administration, and rate of excretion of the agent; the duration of the treatment; drugs used in combination or coincidental with the specific agent; and like factors well known in the medical arts. For example, it is well within the skill of the art to start doses of the agents at levels lower than those required to achieve the desired therapeutic effect and to gradually increase the dosages until the desired effect is achieved.

In a preferred embodiment of the present invention, the antibody-anthracycline conjugate is administered preceded by, concomitantly with, or subsequent to other standard therapies including radiotherapy, surgery or chemotherapy.

In another preferred embodiment, two or more conjugates of an antibody and an anthracycline drug are administered which conjugates target different antigens or different epitopes of the same antigen on the same diseased cells wherein one of the conjugates is a conjugate of the invention. In yet another preferred embodiment, a conjugate of an antibody and an anthracycline drug of the invention is administered, preceded by, concomitantly with, or subsequent to another antibody-based treatment. This additional antibody-based treatment may include the administration of two or more antibody-based treatments, to include naked therapy, where the antibody is administered alone or in combination with another therapeutic-agent that is administered either conjugated or unconjugated to the antibody. The conjugation may utilize the presently disclosed linker or another type linker. When two antibody-based treatments are administered, these treatment are such that whichever antibody is administered second targets a different antigen or a different epitope on the same antigen on diseased cells. The second antibody could also be conjugated with another (different) drug or with a therapeutic isotope, thus providing an antibody-based combination therapy. It is also appreciated that this therapy can be combined, with administration before, simultaneously, or after with cytokines that either enhance the antitumor effects or prevent or mitigate the myelosuppressive effects of the therapeutic conjugates.

Each of the above identified methods of treatment may additionally include the administration of one or more immunomodulators. These immunomodulators may be selected from the group consisting of interferons, cytokines, stem cell growth factors, colony-stimulating factors, lymphotoxins and other hematopoietic factors. The interferon is preferably α-interferon, β-interferon or γ-interferon and the hematopoietic factors may be selected from the group consisting of erythropoietin, thrombopoietin, interleukins (ILs), colony stimulating factors (CSF), granulocyte macrophage-colony stimulating factor (GM-CSF). The interleukin may be selected from the group consisting of IL-1, IL-2, IL-3, IL-6, IL-10, IL-12, IL-18, and IL-21. The immunomodulator or heamatopoietic factor may administered before, during, or after immunconjugate therapy. The immunomodulator is administered to enhance the effectiveness of the administered conjugate of the present invention.

### Kits

The preferred embodiments of the present invention also contemplate kits comprising the conjugate of a monoclonal antibody and an anthracycline drug of the invention in a suitable container. The conjugate includes a linker comprising a hydrazide and a malemide. The monoclonal antibody-anthracycline drug conjugate is provided in a sterile container in liquid, frozen or lyophilized form. The monoclonal antibody-anthracycline drug conjugate can be diluted or reconstituted prior to administration to a patient in need thereof.

In a further embodiment, the conjugate of an anthracycline drug and an antibody of the invention, wherein the anthracycline drug and the antibody are linked via a linker comprising a hydrazide and a maleimide and wherein at least one immunomodulator is further conjugated to the antibody. The conjugate can then be administered to patients in need of therapy as described herein for the conjugate alone or in combination other therapies.

The present invention is illustrated by the following examples, without limiting the scope of the invention.

### EXAMPLES

### General

2-pyrrolino-doxorubicin was prepared using a modified method, based on the original description of Nagy et al. (Proc. Natl. Acad. Sci, U.S.A. 93:2464-2469 (1996)). Morpholino-DOX and cyanomorpholino-DOX were both synthesized from doxorubicin using published methods (Acton et al., J. Med. Chem. 27:638-645 (1984)).

### Example 1: Synthesis of 2 DOX

Synthesis of 2-pyrrolino-doxorubicin (2-PDOX): 4-iodobutyraldehyde: 2-(3-chloropropyl)-1,3-dioxolane (1.3 mL; 10 mM) was dissolved in 200 mL of acetone containing 30 g of sodium iodide (200 mmol; 20-fold excess). The solution is refluxed for 24 h and then evaporated to dryness. The crude mixture is used in the next reaction. Doxorubicin hydrochloride (550 mg, 946 µmol) is dissolved in 6.5 mL of DMF and 3.86 g (19.48 mmol, 20-fold excess) of 4-iodobutyraldehyde is added followed by 500 µL ofN,N-diisopropylethylamine (DIPEA). After five minutes the material is purified by reverse-phase HPLC on a Waters NovaPak C-18 column using a gradient elution. The gradient consisted of 90:10 eluent A to 70:30 eluent B at 75 mL per minute, over 40 minutes, where eluent A is 0.1% trifluoroacetic acid (TFA) and eluent B is 90% acetonitrile containing 0.1% TFA. The identity of the product was confirmed by electrospray mass spectrometry M + H⁺ = 596.

### Example 2: Conjugation of 2-PDOX to the anti-CD22 antibody humanized LL2 (hLL2)

a) Activation of 2-PDOX: 2-PDOX (5.95 mg; 1 x 10⁻⁵ mol) is mixed with a molar equivalent of the commercially available linker 4-[N-maleimidomethyl]cyclohexane-1-carboxylhydrazide (M₂C₂H; Pierce Chemical Co., Rockford, IL) (2.88 mg; 1 x 10⁻⁵ mol) in 0.5 mL of dimethylsulfoxide (DMSO). The reaction mixture is heated at 50-60° C under reduced pressure for thirty minutes. The desired product is purified by preparative RP-HPLC, using a gradient consisting of 0.3 % ammonium acetate and 0.3 % ammonium acetate in 90% acetonitrile, pH 4.4, to separate the desired product from most of the unreacted 2-PDOX (eluting ~0.5 minute earlier) and from unreacted M₂C₂H (eluting much earlier). The amount recovered is estimated by reference to the UV absorbance level of the sample (496 nm), versus a standard solution of 2-PDOX in acetonitrile/ammonium acetate buffer. The maleimide-activated 2-PDOX is frozen and lyophilized, if not used immediately. It is taken up in the minimum amount of DMSO when needed for future reaction with antibodies.

b) Reduction of hLL2 IgG: A 1-mL sample of LL2 antibody (8-12 mg/mL) at 4°C is treated with 100 µL of 1.8 M Tris HCl buffer, followed by three µL of 2-mercaptoethanol. The reduction reaction is allowed to proceed for 10 minutes, and the reduced antibody is purified through two consecutive spin-columns of G-50-80 Sephadex equilibrated in 0.1 M sodium acetate, pH 5.5, containing 1 mM EDTA as anti-oxidant. The product is assayed by UV absorbance at 280 nm, and by Ellman reaction with detection at 410 nm, to determine the number of thiol groups per mole of antibody. These reduction conditions result in the production of approximately 8-12 thiol groups per antibody, corresponding to complete reduction of the antibody's inter-chain disulfide bonds.

c) Conjugation of Activated 2-PDOX to reduced hLL2: The thiol-reduced antibody from b), above, is treated with maleimido-activated 2-PDOX, without allowing the final concentration of DMSO to go above 25% in the aqueous/DMSO mixture. After reaction for 15 minutes at 4° C, the desired product is obtained free of unreacted maleimido-DOX by elution through a G-50-80 spin-column, equilibrated in 0.2 M ammonium acetate, pH 4.4, followed by percolation through a column of SM-2 Bio-Beads equilibrated in the same buffer. The product is analyzed by UV scan at 280 and 496 nm, and the molar ratio of 2-PDOX to mAb is estimated thereby. The absolute 2-PDOX-to-MAb ratio is determined by MALDI-TOF mass spectral analysis. Both UV and MS analyses indicate that a substitution ratio of 7-8 units of 2-PDOX per mole of hLL2 antibody, is obtained under this set of reaction conditions. Upon analysis by size-exclusion HPLC (GF-250 column, Bio-Rad, Hercules CA) run at 1 mL/minute in 0.2 M acetate buffer, pH 5.0, with a UV detector set at 496 nm, essentially all the detected peak elutes near the retention time of the LL2 antibody. This indicates that very little free drug is present in the product. Samples of 2-PDOX-hLL2 conjugate are aliquoted into single fractions, typically of 0.1-1.0 mg, and frozen for future use, or, alternatively, they are lyophilized. They are defrosted or reconstituted, as needed, for further testing.

### Example 3: Conjugation of 2-PDOX to the anti-CD74 antibody humanized LL1 (hLL1)

a) Activation of 2-PDOX: 2-PDOX (5.95 mg; 1 x 10⁻⁵ mol) is mixed with a molar equivalent of the commercially available linker 4-[N-maleimidomethyl]cyclohexane-1-carboxylhydrazide (M₂C₂H; Pierce Chemical Co., Rockford, IL) (2.88 mg; 1 x 10⁻⁵ mol) in 0.5 mL of DMSO. The reaction mixture is heated at 50-60° C under reduced pressure for thirty minutes. The desired product is purified by preparative RP-HPLC, using a gradient consisting of 0.3 % ammonium acetate and 0.3 % ammonium acetate in 90% acetonitrile, pH 4.4, to separate the desired product from most of the unreacted 2-PDOX (eluting ∼ 0.5 minute earlier) and from unreacted M₂C₂H (eluting much earlier). The amount recovered is estimated by reference to the UV absorbance level of the sample (496 nm), versus a standard solution of 2-PDOX in acetonitrile/ammonium acetate buffer. The maleimide-activated 2-PDOX is frozen and lyophilized, if not used immediately. It is taken up in the minimum amount of dimethylformamide (DMF) or DMSO when needed for future reaction with antibodies.

b) Reduction of hLL1 IgG: A 1-mL sample of hLL1 antibody (8-12 mg/mL) at 4°C is treated with 100 µl of 1.8 M Tris HCl buffer, followed by three µL of 2-mercaptoethanol. The reduction reaction is allowed to proceed for 10 minutes, and the reduced antibody is purified through two consecutive spin-columns of G-50-80 Sephadex equilibrated in 0.1 M sodium acetate, pH 5.5, containing 1 mM EDTA as anti-oxidant. The product is assayed by UV absorbance at 280 nm, and by Ellman reaction with detection at 410 nm, to determine the number of thiol groups per mole of antibody. These reduction conditions result in the production of approximately eight-to-ten thiol groups per antibody, corresponding to complete reduction of the antibody's inter-chain disulfide bonds.

c) Conjugation of Activated 2-PDOX to reduced hLL1 : The thiol-reduced antibody from b), above, is treated with maleimido-activated 2-PDOX from a) above, with the final concentration of DMSO of 15% in the aqueous/DMSO mixture. After reaction for 15 minutes at 4°C, the desired product is obtained free of unreacted maleimido-DOX by elution through a G-50-80 spin-column, equilibrated in 0.2 M ammonium acetate, pH 4.4, followed by percolation through a column of SM-2 Bio-Beads equilibrated in the same buffer. The product is analyzed by UV scan at 280 and 496 nm, and the molar ratio of 2-PDOX to mAb is estimated thereby. The absolute 2-PDOX-to-MAb ratio is determined by MALDI-TOF mass spectral analysis. Both UV and MS analyses indicate that a substitution ratio of 7-8 units of 2-PDOX per mole of hLL1 antibody, is obtained under this set of reaction conditions. Upon analysis by size-exclusion HPLC (GF-250 column, Bio-Rad, Hercules CA) run at 1 mL/minute in 0.2 M acetate buffer, pH 5.0, with a UV detector set at 496 nm, essentially one detected peak elutes near the retention time of the hLL1 antibody. This indicates that very little free or no drug is present in the product. Samples of 2-PDOX-hLL1 conjugate are aliquoted into single fractions, typically of 0.1-1.0 mg, and frozen for future use, or alternatively they are lyophilized. They are defrosted or reconstituted, as needed, for further testing.

### Example 4: Conjugation of DOX to the anti-CD74 antibody hLL1

a) Activation of DOX: DOX (1 x 10⁻⁵ mol) is mixed with a molar equivalent of the commercially available linker 4-[N-maleimidomethyl] cyclohexane-1-carboxylhydrazide (M₂C₂H; Pierce Chemical Co., Rockford, IL) (2.88 mg; 1 x 10⁻⁵ mole) in 0.5 mL of DMSO. The reaction mixture is heated at 50-60° C for thirty minutes. The desired intermediate, shown below, is purified by preparative RP-HPLC, using a gradient consisting of 0.3 % ammonium acetate and 0.3 % ammonium acetate in 90% acetonitrile, pH 4.4, to separate the desired product from the unreacted DOX (eluting ∼ 0.5 minute earlier) and from unreacted M₂C₂H (eluting much earlier).

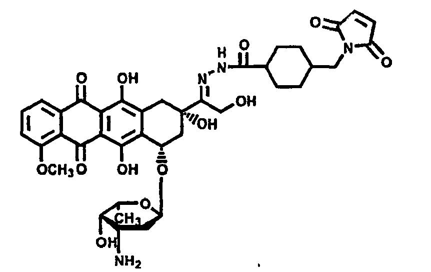

The amount of unreacted DOX is estimated by reference to the UV absorbance level of the sample (496 nm), versus a standard solution of DOX in acetonitrile/ammonium acetate buffer. The maleimide-activated DOX is frozen and lyophilized, if not used immediately. It is taken up in the minimum amount of DMF or DMSO when needed for future reaction with antibodies.

b) Reduction of hLL1 IgG: A 1-mL sample of hLL1 antibody (10 mg/mL) at 4°C is treated with 100 µL of 1.8 M Tris HCl buffer, followed by three µL of 2-mercaptoethanol. The reduction reaction is allowed to proceed for 10 minutes, and the reduced antibody is purified through two consecutive spin-columns of G-50-80 Sephadex equilibrated in 0.1 M sodium acetate, pH 5.5, containing 1 mM EDTA as anti-oxidant. The product is assayed by UV absorbance at 280 nm, and by Ellman reaction with detection at 410 nm, to determine the number of thiol groups per mole of antibody. These reduction conditions result in the production of approximately eight-to-ten thiol groups per antibody, corresponding to complete reduction of the antibody's inter-chain disulfide bonds.

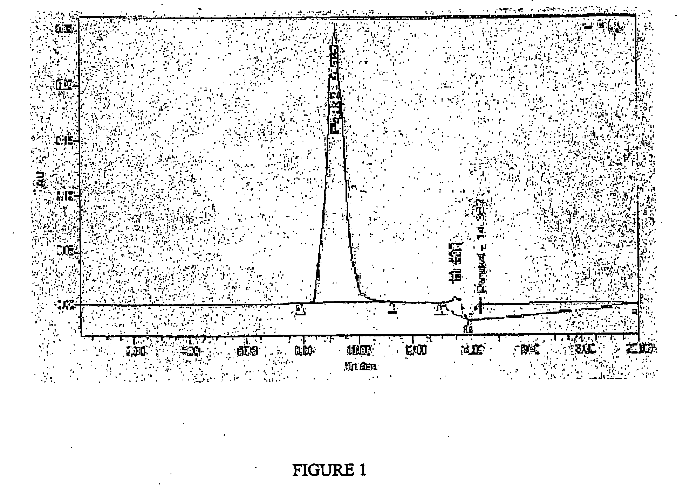

c) Conjugation of activated DOX to reduced hLL1: The thiol-reduced antibody from b), above, is treated with maleimido-activated DOX from a) above, with a final concentration of DMSO of 15% in the aqueous/DMSO mixture. After reaction for 15 minutes at 4°C, the desired product is obtained free of unreacted maleimido-DOX by elution through a G-50-80 spin-column, equilibrated in 0.2 M ammonium acetate, pH 4.4, followed by percolation through a column of SM-2 Bio-Beads equilibrated in the same buffer. The product is analyzed by UV scan at 280 and 496 nm, and the molar ratio of DOX to mAb is estimated thereby. The absolute DOX-to-MAb ratio is determined by MALDI-TOF mass spectral analysis. Both UV and MS analyses indicate that a substitution ratio of 7-8 units of DOX per mole of hLL1 antibody, is obtained under this set of reaction conditions. Upon analysis by size-exclusion HPLC (GF-250 column, Bio-Rad, Hercules CA) run at 1 mL/minute in 0.2 M acetate buffer, pH 5.0, with a UV detector set at 496 nm, essentially one detected peak elutes near the retention time of the hLL1 antibody. The trace (*see* Figure 1; UV detector at 496 nm, set to detect DOX) shows doxorubicin-LL1 conjugate as essentially a single peak at retention time of around nine minutes, without aggregated proteinaceous species or free DOX (retention time around 14 minutes). This indicates that very little free or no drug is present in the product. Samples of DOX-hLL1 conjugate are aliquoted into single fractions, typically of 0.1-1.0 mg, and frozen for future use, or alternatively they are lyophilized. They are defrosted or reconstituted, as needed, for further testing.

### Examples 5: Coupling of doxorubicin to hLL1 and formulation of the dox-hLL1 conjugate

a) Reaction of doxorubicin with SMCC hydrazide

Mix 90 mg of doxorubicin (1.56 x 10⁻⁴ mol) and 60.23 mg of SMCC hydrazide in 13 mL of 1:2 methanol:ethanol (anhydrous), and add 10.4 µL of trifluoroacetic acid. The mixture is allowed to stir for 4 h, in the dark, at room temperature. The reaction solution is then filtered through a 0.22 micron syringe filter into a 100 mL round-bottomed flask. Seventy-five µL of diisopropylethylamine is added and the solvent evaporated on a rotary evaporator at 300° C. The residue is triturated with 4 x 40 mL acetonitrile followed by 1 x 40 mL diethyl ether and dried to a powder on the rotary evaporator under high vacuum. The powder was redissolved in 5 mL anhydrous methanol, re-evaporated to dryness as above, and then stored at -200° C until needed.

b) Reduction of hLL1-IgG with-dithiothreitol

In a 20 mL round bottomed flask are mixed 8.4 mL of hLL1-IgG (10.3 mg/mL, 5.78 x 10⁻⁷ mol), 160 µL of 0.1 M sodium phosphate buffer pH 7.5, 500 µL of 0.2 M EDTA, pH 7.0, and 290 µL of deionized water. The mixture is deoxygenated by cycling solution six times between vacuum and an argon atmosphere. A freshly prepared solution of 40 mM dithiothreitol (DTT) in water (0.015g in 2.4 mL water, 2.3 x 10⁻⁵ mol; 40-fold molar excess to IgG) is deoxygenated by bubbling argon through it for 10 minutes, and 640 µL of this aqueous DTT solution is added to the deoxygenated hLL1 antibody solution. The resulting mixture is incubated at 37° C for 1 hour. The reduced antibody is purified by diafiltration (one 30K filter, under argon, at 4° C), against deoxygenated 10 mM PBS/100 mM L-histidine, pH 7.4, buffer. The buffer is added continuously until total filtrate volume is 300 mL. The volume of the reduced hLL1 solution (hLL1-SH) is reduced to 10 mL.

c) Conjugation of doxorubicin-SMCC to hLL1-SH and purification of conjugate

The activated doxorubicin (1.9 mL, 2.09 x 10⁻⁵ mol, 36-fold excess to IgG) is taken up in dimethylsulfoxide (DMSO) solution and then slowly added to the hLL1-SH antibody solution (40 mL) under argon at room temperature. The final concentration of DMSO is 5%. The reaction is allowed to proceed with gentle stirring for 40 minutes at 4° C. The reaction mixture is loaded onto a BioBeadTM (Bio-Rad, Richmond CA) column (1.5 cm diameter x 34 cm high, equilibrated with 10 mM PBS/100 mM L-histidine, pH 7.4, buffer), and run through at 2 mL/min. The product conjugate is concentrated in an Amicon filtration unit and filtered through a 0.22 micron syringe filter prior to formulation for lyophilization.

d) Conjugate formulation and lyophilization

To 40 mL of the above hLL1-dox solution are added 8 mL of 0.5M mannitol solution in water, and 0.48 mL of 1% polysorbate 20, resulting in final concentrations of 1.64 mg/mL hLL1-dox, 82.5 mM mannitol, and 0.01% polysorbate-20. Samples are lyophilized in 1 mg and 10 mg dox-hLL1 quantities (3 and 10 mL vials, respectively), frozen on dry ice, and lyophilized under vacuum over 48 h. Vials are stoppered under vacuum, and stored sealed at -20° C, in the dark, for future use.

### Example 6: Preparation of morpholino-DOX and cyanomorpholino-DOX conjugates of antibodies

Morpholino-DOX and cyanomorpholino-DOX are prepared by reductive alkylation of doxorubicin with 2,2'-oxy-bis[acetaldehyde], using the procedure of Acton, et al. (J.-Med Chem. 27:638-645 (1984)).

These DOX analogs were coupled with M₂C₂H in the same manner as described above for the DOX and 2-PDOX analogs. Cyanomorpholino-DOX was coupled with 10 % molar excess of the hydrazide in anhydrous methanol (instead of DMSO) overnight at the room temperature. Solvent removal, followed by flash chromatography furnished the hydrazone. Electrospray mass spectral analysis: M+H m/e 872, M+Na 894; M-H 870. In a similar fashion, morpholino-DOX was derivatized to its hydrazone using SMCC-hydrazide using 1.5 equivalent of the reagent in anhydrous methanol for 4 h, and the product was purified by flash chromatography. Electrospray mass spectrum: M+H m/e 847, M-H m/e 845, M+Cl m/e 881.

Interchain disulfide bonds of antibodies were reduced to free thiols as described above in Examples 2-4, to generate disulfide-reduced mAbs, and conjugates were prepared using the same methods as described in section c) of each of Examples 2, 3, and 4. The following mAb conjugates of morpholino-DOX and cyanomorpholino-DOX were prepared:
morpholino-DOX-antibody conjugates:
mRS7 conjugate: drug-to-mAb substitution ratio: 6.4:1.
mMN-14 conjugate: drug-to-mAb substitution ratio: 8.9:1.
cyanomorpholino-DOX-antibody conjugates:
mRS7 conjugate: drug-to-mAb substitution ratio: 5.3:1.
mMN-14 conjugate: drug-to-mAb substitution ratio: 7.0:1.

### Example 7: In Vitro Efficacy of Anthracycline-Antibody Conjugates

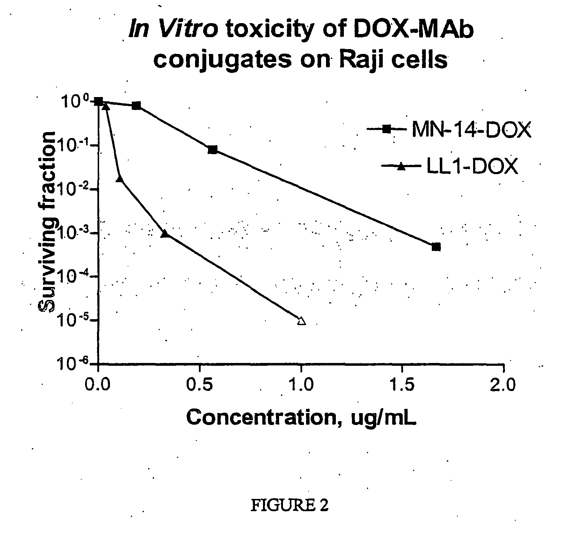

Raji B-lymphoma cells were obtained from the American Type Culture Collection (ATCC, Rockville, MD), and were grown in RPMI 1640 medium containing 12.5% fetal bovine serum (Hyclone, Logan, UT), supplemented with glutamine, pyruvate, penicillin and streptomycin (Life Technologies, Grand Island, NY). Briefly, 3.75 x 10⁵ cells were incubated for 2 days with the indicated concentration of drug-mAb conjugate in 1.5 mL of tissue culture medium in wells of 24-well plates. The cells were then transferred to T25 flasks containing 20 ml of medium, and incubated for up to 21 days, or until the cells had multiplied 16-fold. Viable cell counts using Trypan blue were performed at day 0, day 2, and then every 3-5 days. From the growth rate of untreated cells, the doubling time was calculated, and the Fraction Surviving was calculated from the time required for treated cells to multiply 16-fold, assuming that the doubling time was not affected by treatment. A single remaining viable cell could be readily detected. At a concentration of drug-mAb conjugate of 1 µg/mL the DOX-LL1 conjugate shows a three-orders of magnitude difference in the fraction of surviving cells, in comparison to the DOX-MN-14 conjugate. See Figure 2.

### Example 8: Treatment of Tumor-Bearing Animals with Anthracycline-Antibody Conjugates

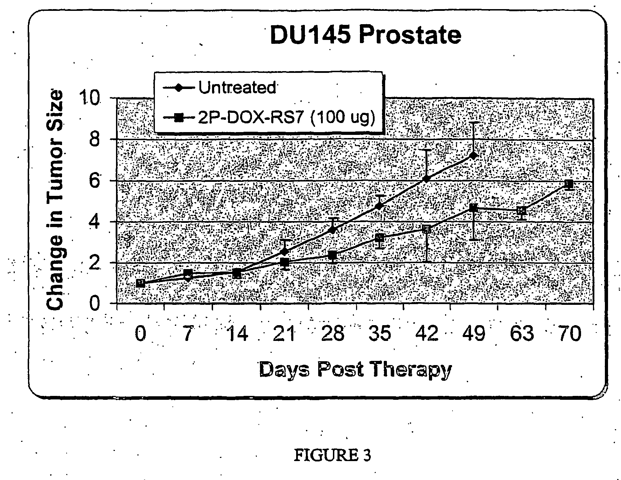

a) Treatment in a solid tumor xenograft model. Groups of athymic nude mice were injected subcutaneously with DU145 human prostate cancer cells. After approximately two weeks, when palpable prostate tumor xenografts had grown in the animals, half were treated with a single dose of the drug-antibody conjugate 2-PDOX-RS7, and half were left untreated (controls). Figure 3, shows the growth of the tumor xenografts in untreated mice versus the growth of xenografts in mice treated with 2-PDOX-RS7. It shows a therapeutic effect for animals treated with the drug-antibody conjugate, in terms of delayed growth of the xenografts.

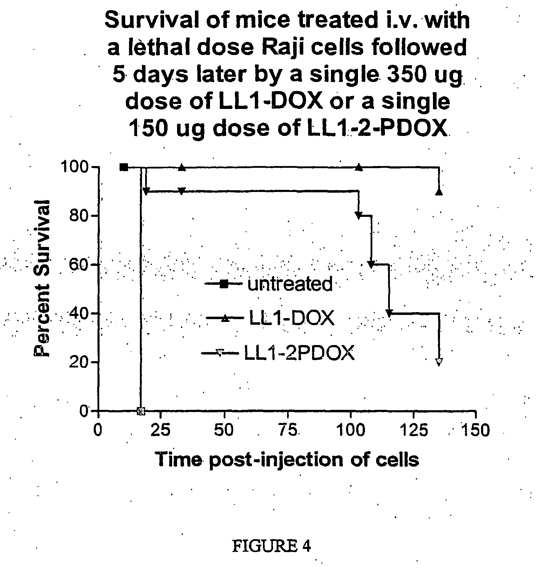

b) Treatment of systemic cancer in an animal model. NCr-SCID mice, in groups of ten animals, were each given an intravenous injection of a suspension of 2.5 x 10⁶ cells of the human Burkitt's B-cell lymphoma cell line, Raji, by tail-vein injection. Five days later, animals were left untreated or treated with single doses of either 350 µg DOX-LL1 or 150 µg 2-PDOX-LL1. Figure 4 shows the result of the experiment. Untreated animals become paralyzed and die at around 23 days post-injection of the Raji cells, from systemic cancer. Animals treated with DOX- and 2-PDOX-conjugates of the LL1 antibody survived over an extended period corresponding to around a four-fold increase in life expectancy for the 2-PDOX-LL1-treated animals, and an even greater increased life expectancy for the DOX-LL1-treated animals.

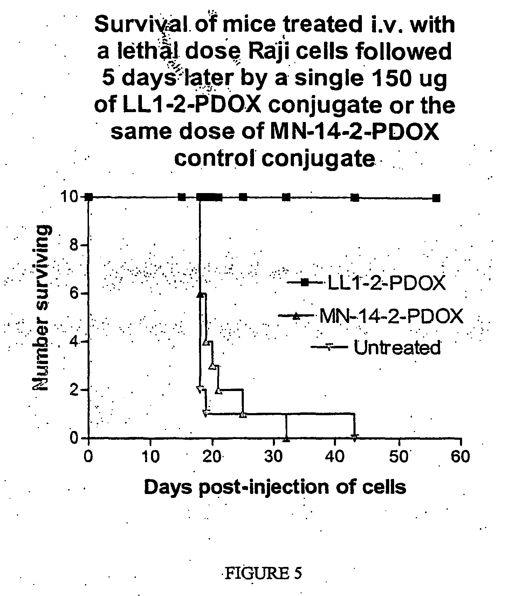

c) Treatment of systemic cancer in an animal model. NCr-SCID mice, in groups of ten animals, were each given an intravenous injection of a suspension of 2.5 x 10⁶ cells of the human Burkitt's B-cell lymphoma cell line, Raji, by tail-vein injection. Five days later, animals were left untreated or treated with single doses of either 150 µg 2-PDOX-LL1 or 150 µg of2-PDOX-MN-14 (non-specific control antibody conjugate). Figure 5 shows the result of the experiment. Untreated animals become paralyzed and die at around 23 days post-injection of the Raji cells, from systemic cancer, as do animals treated with the 2-PDOX-MN-14 conjugate. Animals treated with the 2-PDOX-LL1 antibody conjugate survive over an extended period.

### Cited References

### U.S. Patent Documents:

Arcamone et al., U.S. Patent No. 4,125,607
Greenfield et al., U.S. Patent No. 5,122,368
Jasnaky et al., U.S. Patent No. 6,214,969
Kaneko et al., U.S. Patent No. 5,349,066
Kaneko et al, U.S. Patent No. 5,137,877
McKenzie et al., U.S. Patent No. 5,798,097
King et al., U.S. Patent No. 6,307,026
King et al., U.S. Patent No. 5,824,805
King et al., U.S. Patent No. 5,162,512
Moreland et al., U.S. Patent No. 5,241,078
Schally et al., U.S. Patent No. 6,184,374
Schally et al., U.S. Patent No. 5,843,903
Willner et al., U.S. Patent No. 5,708,146
Willner et al., U.S. Patent No. 5,622,929
Willner et al., U.S. Patent No. 5,606,017

### Other References:

Anthracycline Antibiotics: New Analogs, Methods of Delivery, and Mechanisms of Action. ACS Symposium Series 574, W. Priebe, editor, Publ. American Chemical Society, 1995.
Acton et al., J. Med. Chem., 27:638-645, 1984.
Arencibia et al., Anticancer Drugs, 12:71-78, 2001.
Benali et al., Proc. Natl. Acad. Sci. U.S.A., 97:9180-9185, 2000.
Chastzistamou et al., Clin. Cancer Res., 6:4158-4165, 2000.
Denmeade et al., Cancer Res., 58:2537-40, 1998.
Dubowchik et al., Bioconjug. Chem., 13:855-869,2002.
Halmos et al., Cancer Lett., 136:129-136, 1999.
Hansen et al., Biochem J., 320:393-300, 1996.
Kahan et al., Breast Cancer Res. Treat., 59:255-262, 2000.
Kahan et al., Int. J. Cancer, 82:592-598, 1999.
Kahan et al., Cancer 85:2608-2615, 1999.
Kiaris et al., Eur. J. Cancel 37:620-628, 2001.
Kiaris et al., Br. J. Cancer, 81:966-971, 1999.
Koppan et al., Cancer Res., 58:4132-4137, 1998.
Krebs et al., Cancer Res., 60:4194-4199, 2000.
Michel et al., Clin. Cancer Res., 8:2632-2639, 2002.
Miyazaki et al., Am. J. Obstet. Gynecol. 180:1095-103, 1999.
Miyazaki et al., J. Natl. Cancer Inst., 89:1803-1809, 1997.
Mosure et al., Cancer Chemother. Pharmacol., 40:251-258, 1997.
Nagy et al., Proc. Natl. Acad. Sci. U.S.A., 97:829-834, 2000.
Nagy et al., Proc. Natl. Acad. Sci. U.S.A., 95:1794-1799, 1998.
Nagy et al., Proc. Natl. Acad. Sci. U S A., 94:652-656, 1997.
Nagy et al., Proc. Natl. Acad. Sci. U.S.A., 93:7269-7273, 1996.
Nagy et al., Proc. Natl. Acad. Sci, U.SA., 93:2464-2469, 1996.
Ong et al. Immunology, 98:296-302, 1999.
Pawlyk-Byczkowska et al., Cancer Res., 49:4568-4577, 1989.
Plonowski et al., Int. J. Cancer, 88:652-657, 2000.
Plonowski et al., Cancer Res., 60:2996-3001, 2000.
Plonowski et al., Cancer Res., 59:1947-1953, 1999.
Roche et al., Proc. Natl. Acad. Sci. U.S.A., 90:8581-8585, 1993.
Schally et al., Clin. Cancer Res., 7:2854-2861, 2001.
Schally et al., Prostate, 45:158-166, 2000.
Schally et al., Eur. J. Endocrinol., 141:1-14, 1999.
Shih et al. Cancer Immunol. Immunother., 49:208-216, 2000.
Suzawa et al., J. Contr. Release, 79:229-242, 2002.
Westphalen et al., Int. J. Oncol. 17:1063-1069, 2000.
Wraight et al., J. Biol. Chem., 265:5787-5792, 1990.

## Claims

1. A conjugate of an anthracycline drug and an antibody, wherein said anthracycline drug and said antibody are linked via a linker comprising a hydrazide and a maleimide,
and
said antibody is an internalizing antibody directed against CD74 and wherein the antibody-anthracycline conjugate is internalized into target cells.

2. The conjugate of claim 1, wherein said linker is 4- (N-maleimidomethyl) cyclohexane-1-carboxyl hydrazide.

3. The conjugate of claim 1 having the formula: 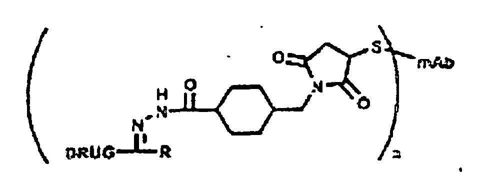 , wherein R is either a hydrogen atom or a C₁-C₆ alkyl group optionally substituted with a hydroxyl group.

4. The conjugate of claim 1, wherein said conjugate targets carcinomas, lymphomas, leukemias, or skin cancers.

5. The conjugate of claim 4, wherein said skin cancer is a melanoma.

6. The conjugate of claim 1, wherein the antibody is LL1.

7. The conjugate of claim 1, wherein said linker is attached to a reduced disulfide bond on the antibody.

8. The conjugate of claim 1, wherein said anthracycline drug is selected from the group consisting of daunorubicin, doxorubicin, epirubicin, 2-pyrrolinodoxorubicin, morpholino-doxorubicin, and cyanomorpholino-doxorubicin.

9. The conjugate of claim 8, wherein said anthracycline drug is linked to the antibody through the 13-keto moiety.

10. The conjugate of claim 7, wherein said reduced disulfide bond is an interchain disulfide bond on the antibody.

11. The conjugate of claim 1, wherein the antibody is murine, chimeric, primatized, humanized, or human.

12. The conjugate of claim 11, wherein the antibody is an antigen-binding fragment of an IgG.

13. The conjugate of claim 1, wherein there are 6-10 molecules of anthracycline drug per molecule of antibody.

14. The conjugate of claim 1, wherein the antibody-anthracycline conjugate is internalized into target cells, and the antigen is then re-expressed on the cell surface.

15. The conjugate of claim 1, wherein the overall electric charge of the antibody is not changed.

16. The conjugate of claim 1, wherein the anthracycline drug bears an alkylating moiety.

17. The conjugate of claim 16, wherein the alkylating moiety is an enamine.

18. The conjugate of claim 17, wherein the anthracycline drug is 2-pyrrolino-doxorubicin.

19. A process for producing the conjugate of claim 1, wherein the linker is first conjugated to the anthracycline drug, thereby producing an anthracycline drug-linker conjugate, and wherein said anthracycline drug-linker conjugate is subsequently conjugated to a thiol-reduced monoclonal antibody or antibody fragment.

20. The process of claim 19, wherein the anthracycline drug-linker conjugate is not purified prior to conjugation to the thiol-reduced monoclonal antibody or antibody fragment.

21. A process for preparing the conjugate of claim 1, wherein secondary reactive functional groups on the anthracycline drug are not compromised.

22. A process for preparing the conjugate of claim 1, wherein alkylating groups on the anthracycline drugs are not compromised.

23. A process for preparing the conjugate of claim 1, wherein said anthracycline drug is 2-pyrrolino-doxorubicin, morpholino-doxorubicin or cyanomorpholino-doxorubicin.

24. Use of a conjugate of an antibody and an anthracycline drug of claim 1 for the manufacture of a medicament to be used in a mammal.

25. Use of claim 24, wherein said mammal is a human.

26. Use of claim 24, wherein the antibody-drug conjugate is administered intravenously, intra-peritoneally, intra-arterially, intra-thecally, intra-vesically, or intra- tumorally.

27. Use of claim 24, wherein the antibody-drug conjugate is given as a bolus or as an infusion.

28. Use of claim 24, wherein the antibody-drug conjugate is given on a repeat and/or cyclical basis.

29. of claim 24, wherein the mammal is suffering from a cancer.

30. Use of claim 24, wherein the mammal is suffering from carcinoma, lymphoma, leukemia or skin cancer.

31. Use of claim 24, wherein the mammal is suffering from a B- or T-cell cancer.

32. Use of claim 31, wherein the mammal is suffering from non-Hodgkin's lymphoma, Hodgkin's disease, lymphatic leukemia, myeloid leukemia or multiple myeloma.

33. Use of claim 30, wherein the mammal is suffering from melanoma.

34. Use of any one of claims 24 to 33, wherein the antibody-anthracycline conjugate is administered preceded by, concomitantly with, or subsequent to other standard therapies.

35. Use of claim 34, wherein said standard therapy is selected from the group consisting of radiotherapy, surgery and chemotherapy.

36. Use of two or more conjugates of an antibody and an anthracycline drug that target different antigens or different epitopes of the same antigen on the same diseased cells for the manufacture of a medicament for treating disease in a mammal, whereby one of the conjugates is a conjugate as defined in claim 1.

37. Use of a conjugate of an antibody and an anthracycline drug as defined in claim 1 for the manufacture of a medicament for treating disease in a mammal, wherein the conjugate of an antibody and an anthracycline drug is administered preceded by, concomitantly with, or subsequent to a second antibody-based treatment, wherein the second antibody in the second antibody-based treatment targets a different antigen or a different epitope on the same antigen on diseased cells than the antibody in the conjugate.

38. The conjugate of claim 1, wherein said antibody is a monoclonal antibody.

39. The conjugate of claim 1, wherein said antibody is an antigen-binding antibody fragment.

40. The conjugate of claim 1, wherein said antibody is a multispecific antibody fusion protein.

41. The conjugate of claim 40, wherein said antibody fusion protein comprises two or more of the same or different natural or synthetic antibody, single-chain antibody or antibody fragment segments with the same or different specificities, wherein said antibody or antibody fragment is selected from the group consisting of LL2, M195, MN-3, RS7, 17- 1A, RS11, PAM-4, KC4, BrE3, MN-14, Mu-9, Immu 31, CC49, Tn antibody, J591, Le(y) antibody and G250.

42. A kit comprising a conjugate of a monoclonal antibody and an anthracycline drug as defined in claim 1 in a suitable container, wherein said anthracycline drug and said antibody are linked via a linker comprising a hydrazide and a maleimide.

43. The kit of claim 42, wherein the monoclonal antibody-anthracycline drug conjugate is provided in a sterile container in liquid, frozen or lyophilized form.

44. The kit of claim 43, wherein the monoclonal antibody-anthracycline drug conjugate is diluted or reconstituted for patient administration.

45. Use of claim 34, wherein the method further comprises administering one or more immunomodulators.

46. Use of claim 35, wherein the method further comprises administering one or more immunomodulators.

47. Use of any one of claims 36 or 37, wherein the method further comprises administering one or more immunomodulators.

48. Use of claim 45, wherein said immunomodulators are selected from the group consisting of interferons, cytokines, stem cell growth factors, colony-stimulating factors, lymphotoxins and other hematopoietic factors.

49. Use of claim 48, wherein said interferon is α-interferon, β-interferon or γ-interferon.

50. Use of claim 48, wherein said hematopoietic factors are selected from the group consisting of interleukins, colony stimulating factors, granulocyte macrophage-colony stimulating factor.

51. Use of claim 50, wherein said interleukin is selected from the group consisting of IL-1, IL-2, IL-3, IL-6, IL-10, IL-12, IL-18, and IL-21.

52. Use of claim 48, wherein said hematopoietic factor is selected from the group consisting of erythropoietin, thrombopoietin, G-CSF and GM-CSF.

53. Use of claim 48 wherein said immunomodulator or hematopoietic factor is given before, during, or after immunconjugate therapy.

54. Use of claim 48, wherein said immunomodulator enhances the effectiveness of said conjugate.

55. Use of claim 46, wherein said immunomodulators are selected from the group consisting of interferons, cytokines, stem cell growth factors, colony-stimulating factors, lymphotoxins and other hematopoietic factors.

56. Use of claim 55, wherein said interferon is α-interferon, β-interferon or γ-interferon.

57. Use of claim 55, wherein said hematopoietic factors are selected from the group consisting of interleukins, colony stimulating factors, granulocyte macrophage-colony stimulating factor.

58. Use of claim 57, wherein said interleukin is selected from the group consisting of IL-1, IL-2, IL-3, IL-6, IL-10, IL-12, IL-18, and IL-21.

59. Use of claim 55, wherein said hematopoietic factor is selected from the group consisting of erythropoietin, thrombopoietin, G-CSF and GM-CSF.

60. Use of claim 55, wherein said immunomodulator or hematopoietic factor is given before, during, or after immunconjugate therapy.

61. Use of claim 55, wherein said immunomodulator enhances the effectiveness of said conjugate.

62. Use of claim 47, wherein said immunomodulators are selected from the group consisting of interferons, cytokines, stem cell growth factors, colony-stimulating factors, lymphotoxins and other hematopoietic factors.

63. Use of claim 62, wherein said interferon is α-interferon, β-interferon or γ-interferon.

64. Use of claim 62, wherein said hematopoietic factors are selected from the group consisting of interleukins, colony stimulating factors, granulocyte macrophage-colony stimulating factor.

65. Use of claim 64, wherein said interleukin is selected from the group consisting of IL-1, IL-2, IL-3, IL-6, IL-10, IL-12, IL-18, and IL-21.

66. Use of claim 62, wherein said hematopoietic factor is selected from the group consisting of erythropoietin, thrombopoietin, G-CSF and GM-CSF.

67. Use of claim 62, wherein said immunomodulator or heamtopoietic factor is given before, during, or after immunconjugate therapy.

68. Use of claim 62, wherein said immunomodulator enhances the effectiveness of said conjugate.

## Patentansprüche

1. Konjugat aus einem Anthracyclin-Arzneimittel und einem Antikörper, wobei das Antracyclin-Arzneimittel und der Antikörper durch einen Linker verbunden sind, der ein Hydrazid und ein Maleimid umfasst,
und
der Antikörper ein gegen CD74 gerichteter internalisierender Antikörper ist und wobei das Antikörper-Anthracyclin-Konjugat in Zielzellen internalisiert wird.

2. Konjugat nach Anspruch 1, wobei der Linker 4-(N-Maleimidomethyl) cyclohexan-1-carboxyl-hydrazid ist.

3. Konjugat nach Anspruch 1 mit der Formel: 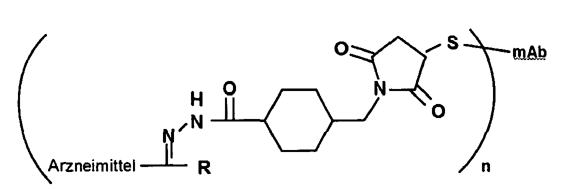 , wobei R entweder ein Wasserstoffatom oder eine optional mit einer Hydroxylgruppe substituierte C₁-C₆ Alkylgruppe ist.

4. Konjugat nach Anspruch 1, wobei das Konjugat Karzinome, Lymphome, Leukemien oder Hautkrebse targetiert.

5. Konjugat nach Anspruch 4, wobei der Hautkrebs ein Melanom ist.

6. Konjugat nach Anspruch 1, wobei der Antikörper LL1 ist.

7. Konjugat nach Anspruch 1, wobei der Linker an eine reduzierte Disulfidbindung auf dem Antikörper gebunden ist.

8. Konjugat nach Anspruch 1, wobei das Anthracyclin-Arzneimittel ausgewählt ist aus der Gruppe bestehend aus Daunorubicin, Doxorubicin, Epirubicin, 2-Pyrrolinodoxorubicin, Morpholino-Doxorubicin und Cyanomorpholino-Doxorubicin.

9. Konjugat nach Anspruch 8, wobei das Anthracyclin-Arzneimittel mit dem Antikörper durch den 13-keto Teil verbunden ist.

10. Konjugat nach Anspruch 7, wobei die reduzierte Disulfidbindung eine Zwischenketten-Disulfidbindung auf dem Antikörper ist.

11. Konjugat nach Anspruch 1, wobei der Antikörper murin, chimär, primatisiert, humanisiert oder human ist.

12. Konjugat nach Anspruch 11, wobei der Antikörper ein Antigen-bindendes Fragment eines IgG ist.

13. Konjugat nach Anspruch 13, wobei 6-10 Anthracyclin-Arzneimittel-Moleküle pro Antikörpermolekül vorhanden sind.

14. Konjugat nach Anspruch 1, wobei das Antikörper-Anthrayclin-Konjugat in Zielzellen internalisiert wird und das Antigen dann auf der Zelloberfläche re-exprimiert wird.

15. Konjugat nach Anspruch 1, wobei die elektrische Gesamtladung des Antikörpers nicht verändert ist.

16. Konjugat nach Anspruch 1, wobei das Anthracyclin-Arzneimittel einen alkylierenden Teil trägt.

17. Konjugat nach Anspruch 16, wobei der alkylierende Teil ein Enamin ist.

18. Konjugat nach Anspruch 17, wobei das Anthracyclin-Arzneimittel 2-Pyrrolino-Doxorubicin ist.

19. Verfahren zur Herstellung des Konjugates nach Anspruch 1, wobei der Linker zuerst mit dem Anthracyclin-Arzneimittel konjugiert wird, wodurch ein Anthracyclin-Arzneimittel-Linker-Konjugat hergestellt wird und wobei das Anthracyclin-Arzneimittel-Linker-Konjugat anschließend mit einem thiol-reduzierten monoklonalen Antikörper oder Antikörper-Fragment konjugiert wird.

20. Verfahren nach Anspruch 19, wobei das Anthracyclin-Arzneimittel-Linker-Konjugat vor der Konjugation mit dem thiol-reduzierten monoklonalen Antikörper oder Antikörper-Fragment nicht gereinigt wird.

21. Verfahren zur Herstellung des Konjugates nach Anspruch 1, wobei sekundäre reaktive funktionelle Gruppen auf dem Anthracyclin-Arzneimittel nicht beeinträchtigt werden.

22. Verfahren zur Herstellung des Konjugates nach Anspruch 1, wobei alkylierende Gruppen auf den Anthracyclin-Arzneimitteln nicht beeinträchtigt werden.

23. Verfahren zur Herstllung des Konjugates nach Anspruch 1, wobei das Anthracyclin-Arzneimittel 2-Pyrrolino-Doxorubicin, Morpholino-Doxorubicin oder Cyanomorpholino-Doxorubicin ist.

24. Verwendung eines Konjugates aus einem Antikörper und einem Anthracyclin-Arzneimittel nach Anspruch 1 zur Herstellung eines Medikaments zur Verwendung in einem Säugetier.

25. Verwendung nach Anspruch 24, wobei das Säugetier ein Mensch ist.

26. Verwendung nach Anspruch 24, wobei das Antikörper-Arzneimittel-Konjugat intravenös, intraperitoneal, intraarteriell, intrathekal, intravesikal oder intratumoral verabreicht wird.

27. Verwendung nach Anspruch 24, wobei das Antikörper-Arzneimittel-Konjugat als ein Bolus oder als eine Infusion gegeben wird.

28. Verwendung nach Anspruch 24, wobei das Antikörper-Arzneimittel-Konjugat wiederholt und/oder zyklisch gegeben wird.

29. Verwendung nach Anspruch 24, wobei das Säugetier an einem Krebs leidet.

30. Verwendung nach Anspruch 24, wobei das Säugetier an Karzinom, Lymphom, Leukämie oder Hautkrebs leidet.

31. Verwendung nach Anspruch 24, wobei das Säugetier an einem B- oder T-Zell-Krebs leidet.

32. Verwendung nach Anspruch 31, wobei das Säugetier an non-Hodgkin-Lymphom, Hodgkin'scher Krankheit, lymphatischer Leukämie, myeloischer Leukämie oder multiplem Myelom leidet.

33. Verwendung nach Anspruch 30, wobei das Säugetier an Melanom leidet.

34. Verwendung nach einem der Ansprüche 24 bis 33, wobei das Antikörper-Anthracyclin-Konjugat vor, gemeinsam mit oder im Nachgang zu anderen Standardtherapien verabreicht wird.

35. Verwendung nach Anspruch 34, wobei die Standardtherapie ausgewählt ist aus der Gruppe bestehend aus Radiotherapie, Chirurgie und Chemotherapie.

36. Verwendung von zwei oder mehr Konjugaten aus einem Antikörper und einem Anthracyclin-Arzneimittel, die verschiedene Antigene oder verschiedene Epitope des gleichen Antigens auf den gleichen erkrankten Zellen targetieren, zur Herstellung eines Medikaments zur Behandlung einer Krankheit in einem Säugetier, wobei eines der Konjugate ein wie in Anspruch 1 definiertes Konjugat ist.

37. Verwendung eines Konjugates aus einem Antikörper und einem Anthracyclin-Arzneimittel wie in Anspruch 1 definiert, zur Herstellung eines Medikaments zur Behandlung einer Krankheit in einem Säugetier, wobei das Konjugat aus einem Antikörper und einem Anthracyclin-Arzneimittel vor, gemeinsam mit oder im Nachgang zu einer zweiten Antikörper-basierten Behandlung verabreicht wird, wobei der zweite Antikörper in der zweiten Antikörper-basierten Behandlung ein anderes Antigen oder ein anderes Epitop auf dem gleichen Antigen auf einer erkrankten Zelle als der Antikörper in dem Konjugat targetiert.

38. Konjugat nach Anspruch 1, wobei der Antikörper ein monoklonaler Antikörper ist.

39. Konjugat nach Anspruch 1, wobei der Antikörper ein Antigen-bindendes Antikörper-Fragment ist.

40. Konjugat nach Anspruch 1, wobei der Antikörper ein multispezifisches Antikörper-Fusionsprotein ist.

41. Konjugat nach Anspruch 40, wobei das Antikörper-Fusionsprotein zwei oder mehr des gleichen oder unterschiedlichen natürlichen oder synthetischen Antikörpers, Einzelketten-Antikörpers oder der Antikörperfragment-Segmente mit den gleichen oder unterschiedlichen Spezifitäten umfasst, wobei der Antikörper oder das Antikörperfragment ausgewählt ist aus der Gruppe bestehend aus LL2, M195, MN-3, RS7, 17-1A, RS11, PAM-4, KC4, BrE3, MN-14, Mu-9, Immu 31, CC49, Tn-Antikörper, J591, Le(y)-Antikörper und G250.

42. Kit umfassend ein Konjugat aus einem monoklonalen Antikörper und einem Anthracyclin-Arzneimittel wie in Anspruch 1 definiert in einem geeigneten Behälter, wobei das Anthracyclin-Arzneimittel und der Antikörper durch einen Linker verbunden sind, der ein Hydrazid und ein Maleimid umfasst.

43. Kit nach Anspruch 42, wobei das monoklonale Antikörper-Anthracyclin-Arzneimittel-Konjugat in einem sterilen Behälter in flüssiger, gefrorener, oder lyophilisierter Form bereitgestellt ist.

44. Kit nach Anspruch 43, wobei das monoklonale Antikörper-Anthracyclin-Arzneimittel-Konjugat zur Patientenverabreichung verdünnt oder rekonstituiert wird.

45. Verwendung nach Anspruch 34, wobei das Verfahren weiter umfasst Verabreichung eines oder mehrerer Immunomodulatoren.

46. Verwendung nach Anspruch 35, wobei das Verfahren weiter umfasst Verabreichung eines oder mehrerer Immunomodulatoren.

47. Verwendung nach einem der Ansprüche 36 oder 37, wobei das Verfahren weiter umfasst Verabreichung eines oder mehrerer Immunomodulatoren.

48. Verwendung nach Anspruch 45, wobei die Immunomodulatoren ausgewählt sind aus der Gruppe bestehend aus Interferonen, Zytokinen, Stammzell-Wachstumsfaktoren, Kolonie-stimmulierenden Faktoren, Lymphotoxinen und anderen hämatopoetischen Faktoren.

49. Verwendung nach Anspruch 48, wobei das Interferon Interferon-α, Interferon-β oder Interferon-γ ist.

50. Verwendung nach Anspruch 48, wobei die hämatopoetischen Faktoren ausgewählt sind aus der Gruppe bestehend aus Interleukinen, Kolonie-stimulierenden Faktoren, Granulozyten-Makrophagen-Kolonie-stimulierendem Faktor.

51. Verwendung nach Anspruch 50, wobei das Interleukin ausgewählt ist aus der Gruppe bestehend aus IL-1, IL-2, IL-3, IL-6, IL-10, IL-12, IL-18 und IL-21.

52. Verwendung nach Anspruch 48, wobei der hämatopoetische Faktor ausgewählt ist aus der Gruppe bestehend aus Erythropoietin, Thrombopoietin, G-CSF und GM-CSF.

53. Verwendung nach Anspruch 48, wobei der Immunomodulator oder hämatopoetische Faktor vor, während oder nach Immunkonjugat-Therapie gegeben wird.

54. Verwendung nach Anspruch 48, wobei der Immunomodulator die Wirksamkeit des Konjugates verstärkt.

55. Verwendung nach Anspruch 46, wobei die Immunomodulatoren ausgewählt sind aus der Gruppe bestehend aus Interferonen, Zytokinen, Stammzell-Wachstumsfaktoren, Kolonie-stimulierenden Faktoren, Lymphotoxinen und anderen hämatopoetischen Faktoren.

56. Verwendung nach Anspruch 55, wobei das Interferon Interferon-α, Interferon-β oder Interferon-γ ist.

57. Verwendung nach Anspruch 55, wobei die hämatopoetischen Faktoren ausgewählt sind aus der Gruppe bestehend aus Interleukinen, Kolonie-stimulierenden Faktoren, Granulozyten-Makrophagen-Kolonie-stimmulierendem Faktor.

58. Verwendung nach Anspruch 57, wobei das Interleukin ausgewählt ist aus der Gruppe bestehend aus IL-1, IL-2, IL-3, IL-6, IL-10, IL-12, IL-18 and IL-21.

59. Verwendung nach Anspruch 55, wobei der hämatopoetische Faktor ausgewählt ist aus der Gruppe bestehend aus Erythropoietin, Thrombopoietin, G-CSF und GM-CSF.

60. Verwendung nach Anspruch 55, wobei der Immunomodulator oder hämatopoetische Faktor vor, während oder nach Immunkonjugat-Therapie gegeben wird.

61. Verwendung nach Anspruch 55, wobei der der Immunomodulator die Wirksamkeit des Konjugates verstärkt.

62. Verwendung nach Anspruch 47, wobei die Immunomodulatoren ausgewählt sind aus der Gruppe bestehend aus Interferonen, Zytokinen, Stammzell-Wachstumsfaktoren, Kolonie-stimulierenden Faktoren, Lymphotoxinen und anderen hämatopoetischen Faktoren.

63. Verwendung nach Anspruch 62, wobei das Interferon Interferon-α, Interferon-β oder Interferon-γ ist.

64. Verwendung nach Anspruch 62, wobei die hämatopoetischen Faktoren ausgewählt sind aus der Gruppe bestehend aus Interleukinen, Kolonie-stimulierenden Faktoren, Granulozyten-Makrophagen-Kolonie-stimulierendem Faktor.

65. Verwendung nach Anspruch 64, wobei das Interleukin ausgewählt ist aus der Gruppe bestehend aus IL-1, IL-2, IL-3, IL-6, IL-10, IL-12, IL-18 and IL-21.

66. Verwendung nach Anspruch 62, wobei der hämatopoetische Faktor ausgewählt ist aus der Gruppe bestehend aus Erythropoietin, Thrombopoietin, G-CSF und GM-CSF.

67. Verwendung nach Anspruch 62, wobei der Immunomodulator oder hämatopoetische Faktor vor, während oder nach Immunkonjugat-Therapie gegeben wird.

68. Verwendung nach Anspruch 62, wobei der der Immunomodulator die Wirksamkeit des Konjugates verstärkt.

## Revendications

1. Conjugué d'un médicament à base d'anthracycline et d'un anticorps, dans lequel ledit médicament à base d'anthracycline et ledit anticorps sont liés via un lieur comprenant un hydrazide et un maléimide,
et
ledit anticorps est un anticorps s'internalisant dirigé contre CD74 et dans lequel le conjugué d'anthracycline-anticorps est internalisé dans les cellules cibles.

2. Conjugué selon la revendication 1, dans lequel ledit lieur est le 4-(N-maléimidométhyl)cyclohexane-1-carboxylhydrazide.

3. Conjugué selon la revendication 1 ayant la formule: 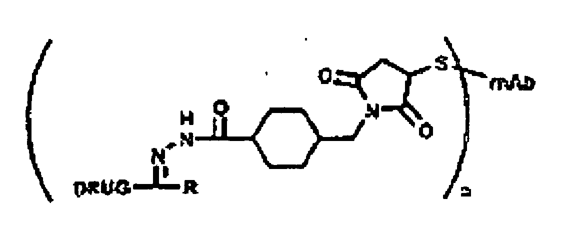 dans lequel R est soit un atome d'hydrogène soit un groupement alkyle en C₁-C₆ facultativement substitué avec un groupement hydroxyle.

4. Conjugué selon la revendication 1, dans lequel ledit conjugué cible des carcinomes, des lymphomes, des leucémies ou des cancers de la peau.

5. Conjugué selon la revendication 4, dans lequel ledit cancer de la peau est un mélanome.

6. Conjugué selon la revendication 1, dans lequel l'anticorps est LL1.

7. Conjugué selon la revendication 1, dans lequel ledit lieur est attaché à une liaison disulfure réduite sur l'anticorps.

8. Conjugué selon la revendication 1, dans lequel ledit médicament à base d'anthracycline est choisi parmi le groupe constitué de la daunorubicine, la doxorubicine, l'épirubicine, la 2-pyrrolino-doxorubicine, la morpholinodoxorubicine et la cyanomorpholino-doxorubicine.

9. Conjugué selon la revendication 8, dans lequel ledit médicament à base d'anthracycline est lié à l'anticorps par la partie 13-céto.

10. Conjugué selon la revendication 7, dans lequel ladite liaison disulfure réduite est une liaison disulfure interchaîne sur l'anticorps.

11. Conjugué selon la revendication 1, dans lequel l'anticorps est un anticorps murin, chimère, primatisé, humanisé ou humain.

12. Conjugué selon la revendication 11, dans lequel l'anticorps est un fragment de liaison à l'antigène d'une IgG.

13. Conjugué selon la revendication 1, dans lequel il y a 6-10 molécules de médicament à base d'anthracycline par molécule d'anticorps.

14. Conjugué selon la revendication 1, dans lequel le conjugué d'anthracycline-anticorps est internalisé dans les cellules cibles, et l'antigène est ensuite réexprimé sur la surface des cellules.

15. Conjugué selon la revendication 1, dans lequel la charge électrique globale de l'anticorps n'est pas changée.

16. Conjugué selon la revendication 1, dans lequel le médicament à base d'anthracycline porte une partie alkylante.

17. Conjugué selon la revendication 16, dans lequel la partie alkylante est une énamine.

18. Conjugué selon la revendication 17, dans lequel le médicament à base d'anthracycline est la 2-pyrrolino-doxorubicine.

19. Procédé de production du conjugué selon la revendication 1, dans lequel le lieur est d'abord conjugué au médicament à base d'anthracycline, produisant de cette façon un conjugué de lieur-médicament à base d'anthracycline, et dans lequel ledit conjugué de lieur-médicament à base d'anthracycline est subséquemment conjugué à un anticorps ou fragment d'anticorps monoclonal thiol réduit.

20. Procédé selon la revendication 19, dans lequel le conjugué de lieur-médicament à base d'anthracycline n'est pas purifié avant conjugaison à l'anticorps ou fragment d'anticorps monoclonal thiol réduit.

21. Procédé de préparation du conjugué selon la revendication 1, dans lequel les groupements fonctionnels réactifs secondaires sur le médicament à base d'anthracycline ne sont pas compromis.

22. Procédé de préparation du conjugué selon la revendication 1, dans lequel les groupements alkylants sur les médicaments à base d'anthracycline ne sont pas compromis.

23. Procédé de préparation du conjugué selon la revendication 1, dans lequel ledit médicament à base d'anthracycline est la 2-pyrrolinodoxorubicine, la morpholinodoxorubicine ou la cyanomorpholino-doxorubicine.

24. Utilisation d'un conjugué d'un anticorps et d'un médicament à base d'anthracycline selon la revendication 1 pour la fabrication d'un médicament à utiliser chez un mammifère.

25. Utilisation selon la revendication 24, dans laquelle ledit mammifère est un être humain.

26. Utilisation selon la revendication 24, dans laquelle le conjugué de médicament-anticorps est administré par voie intraveineuse, intrapéritonéale, intra-artérielle, intrathécale, intravésicale ou intratumorale.

27. Utilisation selon la revendication 24, dans laquelle le conjugué de médicament-anticorps est donné sous forme d'embol ou sous forme de perfusion.

28. Utilisation selon la revendication 24, dans laquelle le conjugué de médicament-anticorps est donné sur une base répétée et/ou cyclique.

29. Utilisation selon la revendication 24, dans laquelle le mammifère souffre d'un cancer.

30. Utilisation selon la revendication 24, dans laquelle le mammifère souffre de carcinome, de lymphome, de leucémie ou de cancer de la peau.

31. Utilisation selon la revendication 24, dans laquelle le mammifère souffre d'un cancer des cellules B ou T.

32. Utilisation selon la revendication 31, dans laquelle le mammifère souffre de lymphome non hodgkinien, de la maladie d'Hodgkin, de leucémie lymphatique, de leucémie myéloïde ou de myélome multiple.

33. Utilisation selon la revendication 30, dans laquelle le mammifère souffre de mélanome.

34. Utilisation selon l'une quelconque des revendications 24 à 33, dans laquelle le conjugué d'anthracycline-anticorps est administré avant, en même temps ou après d'autres thérapies standards.

35. Utilisation selon la revendication 34, dans laquelle ladite thérapie standard est choisie parmi le groupe constitué de la radiothérapie, de la chirurgie et de la chimiothérapie.

36. Utilisation de deux ou de plus de deux conjugués d'un anticorps et d'un médicament à base d'anthracycline qui ciblent des antigènes différents ou des épitopes différents du même antigène sur les mêmes cellules malades pour la fabrication d'un médicament pour traiter la maladie chez un mammifère, où un des conjugués est un conjugué selon la revendication 1.

37. Utilisation d'un conjugué d'un anticorps et d'un médicament à base d'anthracycline selon la revendication 1 pour la fabrication d'un médicament pour traiter la maladie chez un mammifère, dans laquelle le conjugué d'un anticorps et d'un médicament à base d'anthracycline est administré avant, en même temps ou après un traitement à base d'un second anticorps, où le second anticorps dans le traitement à base d'un second anticorps cible un antigène différent ou un épitope différent sur le même antigène sur les cellules malades que l'anticorps dans le conjugué.

38. Conjugué selon la revendication 1, dans lequel ledit anticorps est un anticorps monoclonal.

39. Conjugué selon la revendication 1, dans lequel ledit anticorps est un fragment d'anticorps de liaison à l'antigène.

40. Conjugué selon la revendication 1, dans lequel ledit anticorps est une protéine de fusion d'anticorps multispécifique.

41. Conjugué selon la revendication 40, dans lequel ladite protéine de fusion d'anticorps comprend deux ou plus de deux des anticorps naturels ou synthétiques identiques ou différents, des segments d'anticorps ou de fragments d'anticorps simple chaîne avec des spécificités identiques ou différentes, où l'anticorps ou le fragment d'anticorps est choisi parmi le groupe constitué de LL2, M195, MN-3, RS7, 17-1A, RS11, PAM-4, KC4, BrE3, MN-14, Mu-9, Immu 31, CC49, anticorps Tn, J591, anticorps Le(y) et G250.

42. Trousse comprenant un conjugué d'un anticorps monoclonal et d'un médicament à base d'anthracycline selon la revendication 1 dans un conteneur approprié, dans laquelle ledit médicament à base d'anthracycline et ledit anticorps sont liés via un lieur comprenant un hydrazide et un maléimide.

43. Trousse selon la revendication 42, dans laquelle le conjugué de médicament à base d'anthracycline-anticorps monoclonal est fourni dans un conteneur stérile sous une forme liquide, congelée ou lyophilisée.

44. Kit selon la revendication 43, dans laquelle le conjugué de médicament à base d'anthracycline-anticorps monoclonal est dilué ou reconstitué pour l'administration au patient.

45. Utilisation selon la revendication 34, dans laquelle le procédé comprend en plus l'administration d'un ou de plusieurs immunomodulateurs.

46. Utilisation selon la revendication 35, dans laquelle le procédé comprend en plus l'administration d'un ou de plusieurs immunomodulateurs.

47. Utilisation selon l'une quelconque des revendications 36 ou 37, dans laquelle le procédé comprend en plus l'administration d'un ou de plusieurs immunomodulateurs.

48. Utilisation selon la revendication 45, dans laquelle lesdits immunomodulateurs sont choisis parmi le groupe constitué des interférons, des cytokines, des facteurs de croissance des cellules souches, des facteurs stimulant la formation de colonies, des lymphotoxines et d'autres facteurs hématopoïétiques.

49. Utilisation selon la revendication 48, dans laquelle ledit interféron est l'interféron-α, l'interféron-β ou l'interféron-γ.

50. Utilisation selon la revendication 48, dans laquelle lesdits facteurs hématopoïétiques sont choisis parmi le groupe constitué des interleukines, des facteurs stimulant la formation de colonies, du facteur stimulant la formation de colonies de granulocytes ou de macrophages.

51. Utilisation selon la revendication 50, dans laquelle ladite interleukine est choisie parmi le groupe constitué des IL-1, IL-2, IL-3, IL-6, IL-10, IL-12, IL-18 et IL-21.

52. Utilisation selon la revendication 48, dans laquelle ledit facteur hématopoïétique est choisi parmi le groupe constitué de l'érythropoïétine, de la thrombopoïétine, du G-CSF et du GM-CSF.

53. Utilisation selon la revendication 48, dans laquelle ledit facteur immunomodulateur ou hématopoïétique est donné avant, durant ou après la thérapie de l'immunoconjugué.

54. Utilisation selon la revendication 48, dans laquelle ledit immunomodulateur augmente l'efficacité dudit conjugué.

55. Utilisation selon la revendication 46, dans laquelle lesdits immunomodulateurs sont choisis parmi le groupe constitué des interférons, des cytokines, des facteurs de croissance des cellules souches, des facteurs stimulant la formation de colonies, des lymphotoxines et d'autres facteurs hématopoïétiques.

56. Utilisation selon la revendication 55, dans laquelle ledit interféron est l'interféron-α, l'interféron-β ou l'interféron-γ.

57. Utilisation selon la revendication 55, dans laquelle lesdits facteurs hématopoïétiques sont choisis parmi le groupe constitué des interleukines, des facteurs stimulant la formation de colonies, du facteur stimulant la formation de colonies de granulocytes ou de macrophages.

58. Utilisation selon la revendication 57, dans laquelle ladite interleukine est choisie parmi le groupe constitué des IL-1, IL-2, IL-3, IL-6, IL-10, IL-12, IL-18 et IL-21.

59. Utilisation selon la revendication 55, dans laquelle ledit facteur hématopoïétique est choisi parmi le groupe constitué de l'érythropoïétine, de la thrombopoïétine, du G-CSF et de GM-CSF.

60. Utilisation selon la revendication 55, dans laquelle ledit facteur immunomodulateur ou hématopoïétique est donné avant, durant ou après la thérapie de l'immunoconjugué.

61. Utilisation selon la revendication 55, dans laquelle ledit immunomodulateur augmente l'efficacité dudit conjugué.

62. Utilisation selon la revendication 47, dans laquelle lesdits immunomodulateurs sont choisis parmi le groupe constitué des interférons, des cytokines, des facteurs de croissance des cellules souches, des facteurs stimulant la formation de colonies, des lymphotoxines et d'autres facteurs hématopoïétiques.

63. Utilisation selon la revendication 62, dans laquelle ledit interféron est l'interféron-α, l'interféron-β ou l'interféron-γ.

64. Utilisation selon la revendication 62, dans laquelle lesdits facteurs hématopoïétiques sont choisis parmi le groupe constitué des interleukines, des facteurs stimulant la formation de colonies, du facteur stimulant la formation de colonies de granulocytes ou de macrophages.

65. Utilisation selon la revendication 64, dans laquelle ladite interleukine est choisie parmi le groupe constitué des IL-1, IL-2, IL-3, IL-6, IL-10, IL-12, IL-18 et IL-21.

66. Utilisation selon la revendication 62, dans laquelle ledit facteur hématopoïétique est choisi parmi le groupe constitué de l'érythropoïétine, de la thrombopoïétine, du G-CSF et du GM-CSF.

67. Utilisation selon la revendication 62, dans laquelle ledit facteur immunomodulateur ou hématopoïétique est donné avant, durant ou après la thérapie de l'immunoconjugué.

68. Utilisation selon la revendication 62, dans laquelle ledit immunomodulateur augmente l'efficacité dudit conjugué.
